# EUROPEAN PATENT APPLICATION

(11) **EP 4 652 917 A1**
(43) Date of publication of application: **26.11.2025**
(21) Application number: 23917741.3
(22) Date of filing: 21.12.2023
(51) Int. Cl.: A61B 1/045

(54) **IMAGING SYSTEM AND ELECTRONIC ENDOSCOPE SYSTEM**

(30) Priority: 16.01.2023 JP 2023004384
(71) Applicant: HOYA CORPORATION, Shinjuku-ku Tokyo 160-8347 (JP)
(72) Inventor: HAYASHI Yoshihiro, Tokyo 160-8347 (JP)
(74) Representative: Lucke, Andreas
(86) International application number: PCT/JP2023/045839
(87) International publication number: WO 2024/154530

(57) **Abstract**

According to an aspect of the present invention, provided is an electronic endoscope system including a CMOS image sensor that images an object using a rolling shutter method and a light source device that emits a flash to perform strobe imaging of the object. In the system, the light source device emits a flash such that a period from a flash end time of a certain flash to a flash start time of a next flash is longer than a flash prohibition period during which the emission of the flash is prohibited for at least one frame period. The electronic endoscope system processes a captured image for each frame obtained by the CMOS image sensor on the basis of the emission timing of the flash from the light source device to generate a display image.

## Description

### Technical Field

The present invention relates to stroboscopy performed using an image sensor using a rolling shutter method.

### Background Art

Conventionally, an electronic endoscope system for performing laryngeal stroboscopy is known.

For example, in an electronic endoscope system described in Japanese Patent No. 6196105, pixel signals are continuously read from a CMOS image sensor using a rolling shutter method, and an LED is caused to emit strobe light in synchronization with subject's utterance. Pixel signals of lines read after strobe light emission in a frame being read from the CMOS image sensor when the strobe light (flash) is emitted are held in a frame buffer as a lower image, and in a next frame, pixel signals of the lines above the lower image in the previous frame are directly output to an image signal processing unit, and then the lower image in the frame before being held in the frame buffer is output to the image signal processing unit. Thus, in the strobe imaging with a rolling shutter, an image for one frame captured through the same strobe light emission is obtained without providing a period for strobe exposure.

### Summary of Invention

### Technical Problem

In the electronic endoscope system, there is a problem that a period (flash exposure period) during which strobe light (flash) is emitted needs to be less than a read time of one line of the CMOS image sensor. In a case where the flash exposure period is a short period less than the read time of one line of the CMOS image sensor, it is necessary to increase light intensity of a light source such as an LED in order to obtain sufficient brightness for observing vocal cords, and thus, it is necessary to enhance heat dissipation measures of the light source, or adverse effects such as deterioration in durability of the light source occur. Furthermore, when the gain of the CMOS image sensor is increased without increasing the intensity of the light source, a decrease in the S/N ratio is caused.

An object of the present invention is to increase the degree of freedom in setting the length of a flash exposure period in a case where a flash is emitted to an object and the object is imaged using the rolling shutter method.

### Solution to Problem

According to an aspect of the present disclosure, there is provided an imaging system including:
an image sensor that images an object using a rolling shutter method;
a light source unit that emits a flash to perform strobe imaging of the object, the light source unit emitting the flash such that a period from a flash end time of a certain flash to a flash start time of a next flash is longer than a flash prohibition period during which emission of the flash is prohibited for at least one frame period; and
an image processing unit that processes a captured image for each frame obtained by the image sensor on the basis of an emission timing of the flash from the light source unit to generate a display image.

In a case where the flash is emitted at any line of the image sensor at an exposure start time that is one frame before a current frame,
the image processing unit generate the display image of the current frame by
setting an upper image corresponding to lines above a flash start line that is one frame earlier in the display image of the current frame as a captured image obtained from corresponding lines of the current frame,
setting a lower image corresponding to lines below a flash end line that is one frame earlier in the display image of the current frame as a captured image obtained from corresponding lines that are one frame earlier, and
setting a boundary image from the flash start line to the flash end line in the display image of the current frame as an image obtained by adding the captured image obtained from the corresponding lines of the current frame and the captured image obtained from the corresponding lines that are one frame earlier.

In a case where the flash is not emitted at any line of the image sensor at the exposure start time that is one frame before the current frame,
the image processing unit generates the display image of the current frame by
setting an upper image corresponding to lines above the flash start line that is two frames earlier in the display image of the current frame as a captured image obtained from corresponding lines that are one frame earlier,
setting a lower image corresponding to lines below the flash end line that is two frames earlier in the display image of the current frame as a captured image obtained from corresponding lines that are two frames earlier, and
setting a boundary image from the flash start line to the flash end line in the display image of the current frame as an image obtained by adding the captured image obtained from corresponding lines that are one frame earlier and the captured image obtained from corresponding lines that are two frames earlier.

In the imaging system, the image processing unit may include an amplification processing unit that amplifies a pixel value of each pixel of the display image of the current frame, and
the amplification processing unit may set a gain to be applied to pixels of each line of the boundary image in the display image of the current frame to be larger than a gain to be applied to pixels of each line of images other than the boundary image according to an emission intensity profile corresponding to elapse of time during the flash and a reading period of the image sensor.

The image processing unit may include a filter processing unit that applies a spatial filter to pixel values of pixels included in a predetermined number of upper and lower lines from an adjacent position where the boundary image and the upper image are adjacent to each other and a predetermined number of upper and lower lines from an adjacent position where the boundary image and the lower image are adjacent to each other, for the display image of the current frame.

The image processing unit may include a filter processing unit that applies a spatial filter to pixel values of pixels included in a predetermined number of upper and lower lines from a central position in a line direction of the boundary image, for the display image of the current frame.

The filter processing unit may set filter coefficients of the spatial filter applied to the pixels included in each line of the predetermined number of lines according to an emission intensity profile corresponding to elapse of time during the flash.

The image processing unit includes an interpolation processing unit that performs interpolation processing between a current frame and a past frame that is two or three frames before the current frame, using, as a target line, each line of the boundary image in the display image of the current frame, or each line of a plurality of lines including a predetermined number of lines above an adjacent position where the boundary image and the upper image are adjacent to each other and a predetermined number of lines below an adjacent position where the boundary image and the lower image are adjacent to each other, as well as each line of the boundary image.

In this case,
in a case where the flash is emitted at any line of the image sensor at the exposure start time that is one frame before the current frame, the interpolation processing unit calculates pixel values of pixels included in each target line in the display image of the current frame by performing weighted averaging on pixel values of corresponding pixels in the display image of the current frame and pixel values of corresponding pixels in a captured image that is two frames earlier, and
in a case where the flash is not emitted at any line of the image sensor at the exposure start time that is one frame before the current frame, the interpolation processing unit calculates the pixel values of the pixels included in each target line in the display image of the current frame by performing the weighted average on the pixel values of corresponding pixels in the display image of the current frame and the pixel values of corresponding pixels in the captured image that is three frames earlier.

The interpolation processing unit may set a weight of the weighted average such that the weight for the pixel values of corresponding pixels in the captured image of the past frame is maximized at a center in a line direction of the boundary image, and the weight for the pixel values of corresponding pixels in the captured image of the past frame decreases as a distance from the center increases in a vertical direction.

The interpolation processing unit may set a weight of the weighted average such that the weight for the pixel values of the pixels included in a line at the adjacent position where the boundary image and the upper image are adjacent to each other and/or a line at the adjacent position where the boundary image and the lower image are adjacent to each other is maximized, and the weight for the pixel values of the corresponding pixels of the captured image of the past frame decreases as a distance from the adjacent position increases in a vertical direction.

The interpolation processing unit may set a weight of the weighted average applied to the pixels included in the target line according to an emission intensity profile corresponding to elapse of time during the flash.

According to another aspect of the present disclosure, there is provided an electronic endoscope system including:
a microphone;
a voice detection unit that detects a voice frequency from an audio signal acquired from the microphone; and
the imaging system according to any one of claims 1 to 9,
in which the light source unit emits the flash at a cycle synchronized with the voice frequency detected by the voice detection unit.

### Advantageous Effects of Invention

In the imaging system and electronic endoscope system described above, it is possible to increase the degree of freedom in setting the length of a flash exposure period in a case where a flash is emitted to an object and the object is imaged using the rolling shutter method.

### Brief Description of Drawings

Fig. 1 is a block diagram illustrating an example of a configuration of an electronic endoscope system according to an embodiment.
Fig. 2 is a diagram illustrating a relationship between an operation of a CMOS image sensor and an emission timing of a flash.
Fig. 3 is a timing chart illustrating the operation in generating a composite image when a flash is emitted according to a case where a vocal cord vibration frequency is 1000 Hz in an electronic endoscope system of the embodiment.
Fig. 4 is a timing chart illustrating the operation in generating a composite image when a flash is emitted according to a case where a vocal cord vibration frequency is 250 Hz in an electronic endoscope system of the embodiment.
Fig. 5 is a timing chart illustrating the operation in generating a composite image when a flash is emitted according to a case where a vocal cord vibration frequency is 125 Hz in an electronic endoscope system of the embodiment.
Fig. 6 is a timing chart illustrating the operation in generating a composite image when a flash is emitted according to a case where a vocal cord vibration frequency is 115 Hz in an electronic endoscope system of the embodiment.
Fig. 7 is a timing chart illustrating the operation in generating a composite image when a flash is emitted according to a case where a vocal cord vibration frequency is 63 Hz in an electronic endoscope system of the embodiment.
Fig. 8 is a diagram illustrating a relationship between a vocal cord vibration frequency and a frame rate in an electronic endoscope system of the embodiment.
Fig. 9 is a diagram illustrating the embodiment in which an amplification processing unit and a filter processing unit are added to the system illustrated in Fig. 1.
Fig. 10 is a diagram illustrating an example of a method for improving the image quality of a composite image in an electronic endoscope system according to the embodiment illustrated in Fig. 9.
Fig. 11 is a diagram illustrating an example of spatial filter processing applied to a composite image.
Fig. 12 is a diagram illustrating an example of spatial filter processing applied to a composite image.
Fig. 13 is a diagram illustrating an example of spatial filter processing applied to a composite image.
Fig. 14 is a diagram illustrating the embodiment in which an amplification processing unit and an interpolation processing unit are added to the system illustrated in Fig. 1.
Fig. 15 is a diagram illustrating an example of a method for improving the image quality of a composite image in an electronic endoscope system according to the embodiment illustrated in Fig. 14.
Fig. 16 is a diagram illustrating an example of a method for improving the image quality of a composite image in an electronic endoscope system according to the embodiment illustrated in Fig. 14.
Fig. 17 is a diagram illustrating an example of a method for improving the image quality of a composite image in an electronic endoscope system according to the embodiment illustrated in Fig. 14.
Fig. 18 is a diagram illustrating an example of frame interpolation processing applied to a composite image.
Fig. 19 is a diagram illustrating an example of frame interpolation processing applied to a composite image.
Fig. 20 is a diagram illustrating an example of frame interpolation processing applied to a composite image.
Fig. 21 is a block diagram illustrating an example of a configuration of an electronic endoscope system according to a different embodiment from Fig. 1.
Fig. 22 is a block diagram illustrating an example of a configuration of an electronic endoscope system according to a different embodiment from Fig. 1.

### Description of Embodiments

Hereinafter, an electronic endoscope system of an imaging system according to an embodiment of the present invention will be described in detail with reference to the drawings.

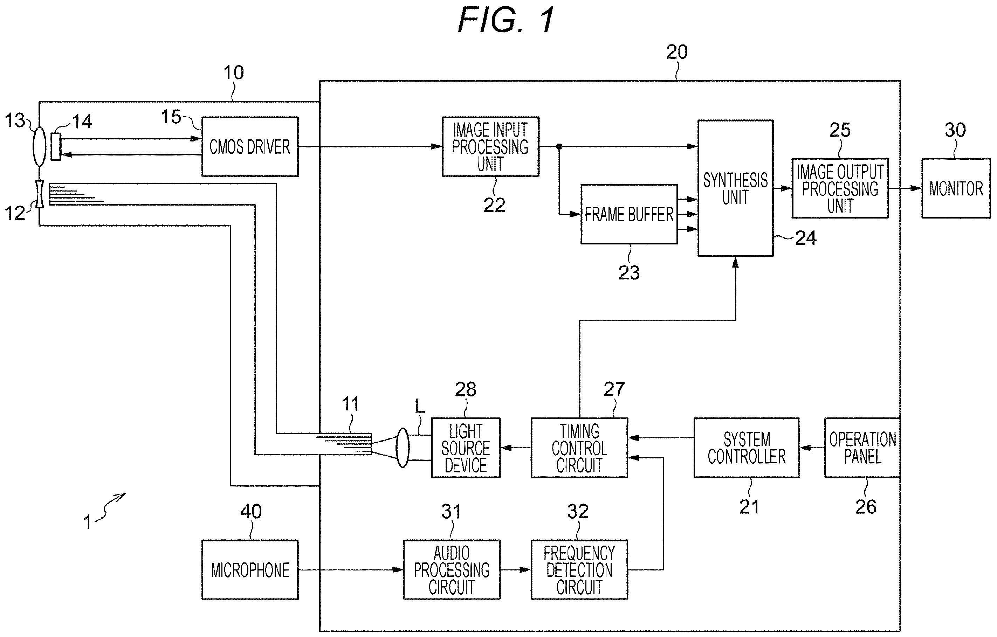

Fig. 1 is a block diagram illustrating an example of a configuration of an electronic endoscope system 1 according to the embodiment. The electronic endoscope system 1 is a system specialized for medical use, and is particularly used for laryngeal stroboscopy. In the laryngeal stroboscopy, the operator observes vocal cords as an object by performing strobe imaging to display an image using a light source device that intermittently emits flashes and an electronic scope.

As illustrated in Fig. 1, the electronic endoscope system 1 according to the embodiment includes an electronic scope (endoscope) 10, a processor 20 incorporating a light source device, a monitor 30, and a microphone 40. The processor 20 is connected to the monitor 30 and the microphone 40.

The processor 20 includes an audio processing circuit 31 and a frequency detection circuit 32.

In a case where strobe imaging of vocal cords is performed, flashes are intermittently turned on (emitted) according to a vocal cord vibration frequency based on a voice generated by a patient. The patient's voice is collected by the microphone 40. The audio processing circuit 31 removes noise and the like from the voice collected by the microphone 40 to obtain a voice waveform suitable for detecting a vocal cord vibration frequency. The frequency detection circuit 32 detects a vocal cord vibration frequency from the voice waveform obtained by the audio processing circuit 31.

The processor 20 includes a system controller 21, an operation panel 26, a timing control circuit 27, and a light source device 28 (an example of a light source unit).

The system controller 21 executes various programs and integrally controls the entire electronic endoscope system 1. The system controller 21 changes operations of the electronic endoscope system 1 and parameters for the operations in accordance with an operator's (observer's) instruction input to the operation panel 26. The system controller 21 supplies clock pulses for adjusting an operation timing of each unit to the corresponding circuits in the electronic endoscope system 1.

Under the control of the system controller 21, the timing control circuit 27 determines the emission timing of the flash according to the vocal cord vibration frequency detected by the frequency detection circuit 32, and sequentially transmits a signal (emission timing signal) indicating the determined emission timing of the flash to the light source device 28. Note that the timing control circuit 27 receives data regarding the length of the emission prohibition period from the system controller 21 at the time of system activation, and determines the emission timing such that the length of the emission prohibition period is maintained. The timing control circuit 27 sequentially transmits the emission timing signal to a synthesis unit 24 in real time.

The flash prohibition period means the shortest period between the flash end time of a certain flash and the flash start time of the next flash. That is, until the flash prohibition period elapses from the flash end time of a certain flash, the emission of the next flash is prohibited. The flash prohibition period is a period of at least one frame duration (one frame cycle) or longer. The reason why the flash prohibition period is set to at least one frame period is to prevent the same line of a CMOS image sensor 14 from being exposed twice or more within one frame period.

The light source device 28 emits a flash L for illuminating an object such as vocal cords in synchronization with the emission timing signal transmitted from the timing control circuit 27. The flash L may be white light, pseudo-white light, or special light in a specific wavelength band. According to the embodiment, the flash L emitted from the light source device 28 is focused onto an incident end face of a light carrying bundle (LCB) 11 by a condenser lens, and enters the LCB 11.

The type of light source of the light source device 28 is not limited, and examples of the light source includes, for example, an LED, a laser diode, and a high brightness lamp (for example, a xenon lamp, a metal halide lamp, a mercury lamp, or a halogen lamp). In the following description, a case where the light source of the light source device 28 is an LED will be described as an example.

The flash L entering the LCB 11 propagates through the LCB 11. The flash L propagating through the LCB 11 is emitted from an emission end face of the LCB 11 disposed at a distal end of the electronic scope 10, and emitted onto the object via a light distribution lens 12. Return light from the object illuminated with the flash L from the light distribution lens 12 forms an optical image on a light reception surface of the CMOS image sensor 14 via an objective lens 13.

The CMOS image sensor 14 is an example of an image sensor configured to image an object using a rolling shutter method. The CMOS image sensor 14 has, for example, a Bayer pattern pixel arrangement, accumulates the optical images formed at pixels on the light reception surface as charge corresponding to the light intensity, and generates and outputs red (R), green (G), and blue (B) image signals. Note that a charge-coupled device (CCD) image sensor or another type of imaging device may be applied instead of the CMOS image sensor 14. The CMOS image sensor 14 may include a complementary color filter.

A CMOS driver 15 provided in the electronic scope 10 controls driving of the CMOS image sensor 14 at a timing synchronized with the frame rate of an image processed by the processor 20 in accordance with the clock pulses supplied from the system controller 21. The CMOS driver 15 performs predetermined processing including A/D conversion on the captured image input from the CMOS image sensor 14 and outputs the processed image to an image input processing unit 22 of the processor 20.

An imaging signal obtained by imaging an object from the CMOS image sensor 14 is input to the CMOS driver 15 at a predetermined frame cycle.

The frame cycle (one frame period) is, for example, 1/120 second, 1/60 second, or 1/30 second, but a case where the frame cycle is 1/60 second will be described below as an example. That is, the length of the flash prohibition period is at least 1/60 second.

The processor 20 includes an image input processing unit 22, a frame buffer 23, a synthesis unit 24, and an image output processing unit 25 in order to generate an image to be displayed on the monitor 30.

The image input processing unit 22 performs predetermined signal processing such as noise reduction processing, demosaicing processing, and matrix operations on the captured image for each frame transmitted from the CMOS driver 15, and transmits the captured image for each frame (captured image of the current frame) to the frame buffer 23 and the synthesis unit 24.

The frame buffer 23 is a memory that buffers the captured image sent from the image input processing unit 22 for each frame. In the embodiment, the frame buffer 23 temporarily stores captured images for three frames that are one, two, and three frames before the current frame. Note that, in a case where frame interpolation processing to be described later is not performed, the frame buffer 23 is only required to temporarily store captured images for two frames that are one and two frames before the current frame.

The synthesis unit 24 generates a composite image by synthesizing the captured images for a plurality of frames stored in the frame buffer 23 on the basis of the emission timing indicated by the emission timing signal received from the timing control circuit 27. A specific example of generating the composite image will be described in detail later.

The image output processing unit 25 processes the composite image generated by the synthesis unit 24 for each frame to generate screen data for monitor display, and converts the generated screen data for monitor display into a predetermined video format signal. The converted video format signal is output to the monitor 30. Thus, an image of the object (vocal cords) is displayed on a display screen of the monitor 30.

The frame buffer 23, the synthesis unit 24, and the image output processing unit 25 constitute an example of an image processing unit of the present invention.

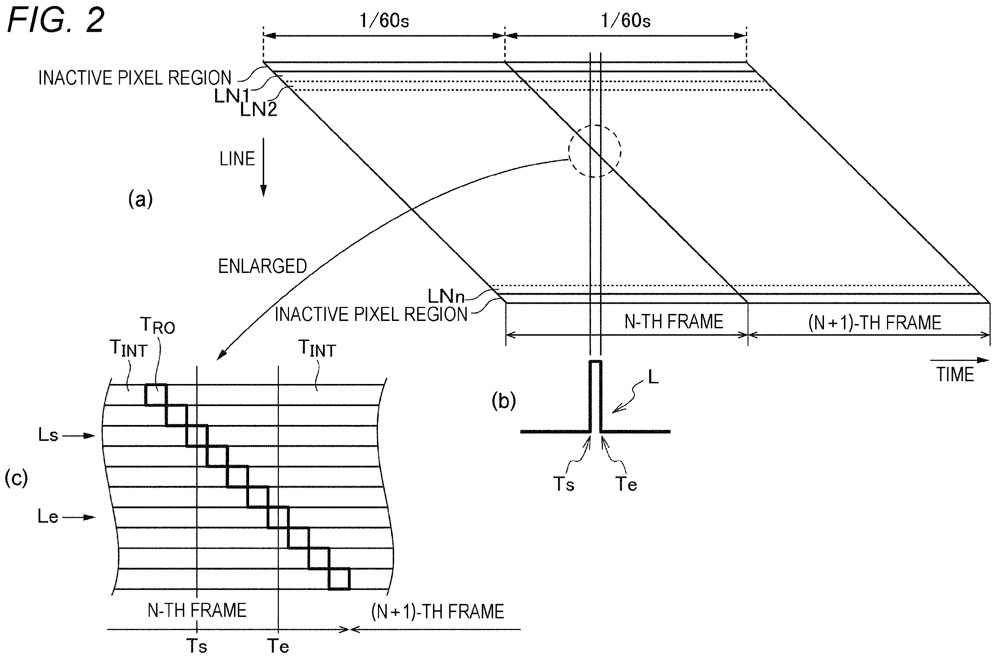

Next, the operation of the CMOS image sensor 14 based on the flash will be described with reference to Fig. 2.

In Fig. 2, (a) illustrates the operation of the CMOS image sensor 14 over a two-frame period (1/60 second + 1/60 second) consisting of an N-th frame and an (N + 1)-th frame, which are consecutive, (b) illustrates the flash L, and (c) illustrates a part of (a) in an enlarged manner.

In the CMOS image sensor 14, exposure in each frame is started by providing a time difference for each of a plurality of lines in the effective pixel region, excluding the inactive pixel region. Note that the inactive pixel region includes one line or a plurality of lines.

As illustrated in Fig. 2(a), in the CMOS image sensor 14, a time difference is provided in the order of lines LN1, LN2, ..., and LNn (the last line of the effective pixel region) included in the effective pixel region excluding the inactive pixel region (the order of the lines from the top to the bottom), and exposure in the N-th frame is started. As illustrated in an enlarged manner in Fig. 2(c), each frame period includes a charge accumulation period T_{INT} in which charge is accumulated in each pixel region of the effective pixel region from the start of exposure, and a signal reading period T_{RO} in which charge accumulation for one frame period ends and a signal corresponding to the accumulated charge is read. The charge accumulation cannot be performed during the signal reading period T_{RO}.

As illustrated in Figs. 2(a) and 2(b), the flash L is light emitted in a short time from a flash start time Ts to a flash end time Te, and is used for charge accumulation of a partial line group of the effective pixel region in the N-th frame and a partial line group of the effective pixel region in the (N + 1)-th frame. The period from the flash start time Ts to the flash end time Te is a flash exposure period. Here, referring to the enlarged drawing of Fig. 2(c), a line corresponding to the flash start time Ts of the flash L is defined as a flash start line Ls, and a line corresponding to the flash end time Te of the flash L is defined as a flash end line Le.

For each line from the line LN1 to the flash start line Ls, the charge generated by the flash L is accumulated in the charge accumulation period T_{INT} of the (N + 1)-th frame. On the other hand, for each line from the flash end line Le to the last line LNn of the effective pixel region, the charge generated by the flash L is accumulated in the charge accumulation period T_{INT} of the N-th frame. For each line from the flash start line Ls to the flash end line Le, the charge generated by part of the flash L is accumulated in the charge accumulation period T_{INT} of the N-th frame, and the charge generated by the remaining part of the flash L is accumulated in the charge accumulation period T_{INT} of the (N + 1)-th frame.

As can be seen from Fig. 2, one display image obtained by the flash L can be obtained on the basis of the composite image obtained by appropriately synthesizing the following upper image, lower image, and boundary image.
· An upper image obtained during the (N + 1)-th frame period from each line from the line LN1 to the flash start line Ls
· A lower image obtained during the N-th frame period from each line from the flash end line Le to the last line LNn of the effective pixel region
· A boundary image between the upper image and the lower image, which are obtained during the n-th and (n + 1)-th frame periods from each line from the flash start line Ls to the flash end line Le

Note that, in the electronic endoscope system 1 of the present embodiment, as long as the emission prohibition period is secured, the emission timing of the flash L can be arbitrarily set, and the flash exposure period can be arbitrarily set. Therefore, there may be a case where the flash L is not emitted at the start time of the charge accumulation period T_{INT} of any line in the effective pixel region of the CMOS image sensor 14 in a certain frame. In consideration of such a case, more specifically, the synthesis unit 24 of the processor 20 generates a composite image as a basis of the display image by classifying the processes into the following processes Pa and Pb.

[Process Pa] In a case where a flash is emitted at any line of the CMOS image sensor 14 at the exposure start time of the frame one frame before the current frame, the synthesis unit 24 generates a composite image of the current frame as follows.

(a-1) An upper image corresponding to lines above the flash start line of the frame that is one frame earlier in the composite image of the current frame is set as a captured image obtained from the corresponding lines of the current frame.

(a-2) A lower image corresponding to lines below the flash end line of the frame that is one frame earlier in the composite image of the current frame is set as a captured image obtained from the corresponding lines of the frame that is one frame earlier.

(a-3) A boundary image between the flash start line and the flash end line in the composite image of the current frame is an image obtained by adding the captured image obtained from the corresponding lines of the current frame and the captured image obtained from the corresponding lines of the frame that is one frame earlier.

[Process Pb] In a case where a flash is not emitted at any line of the CMOS image sensor 14 at the exposure start time of the frame one frame before the current frame, the synthesis unit 24 generates a composite image of the current frame as follows.

(b-1) An upper image corresponding to lines above the flash start line of the frame that is two frames earlier in the composite image of the current frame is set as a captured image obtained from the corresponding lines of the frame that is one frame earlier.

(b-2) A lower image corresponding to lines below the flash end line of the frame that is two frames earlier in the composite image of the current frame is set as a captured image obtained from the corresponding lines of the frame that is two frames earlier.

(b-3) A boundary image between the flash start line and the flash end line in the composite image of the current frame is an image obtained by adding the captured image obtained from the corresponding lines of the frame that is one frame earlier and the captured image obtained from the corresponding lines of the frame that is two frames earlier.

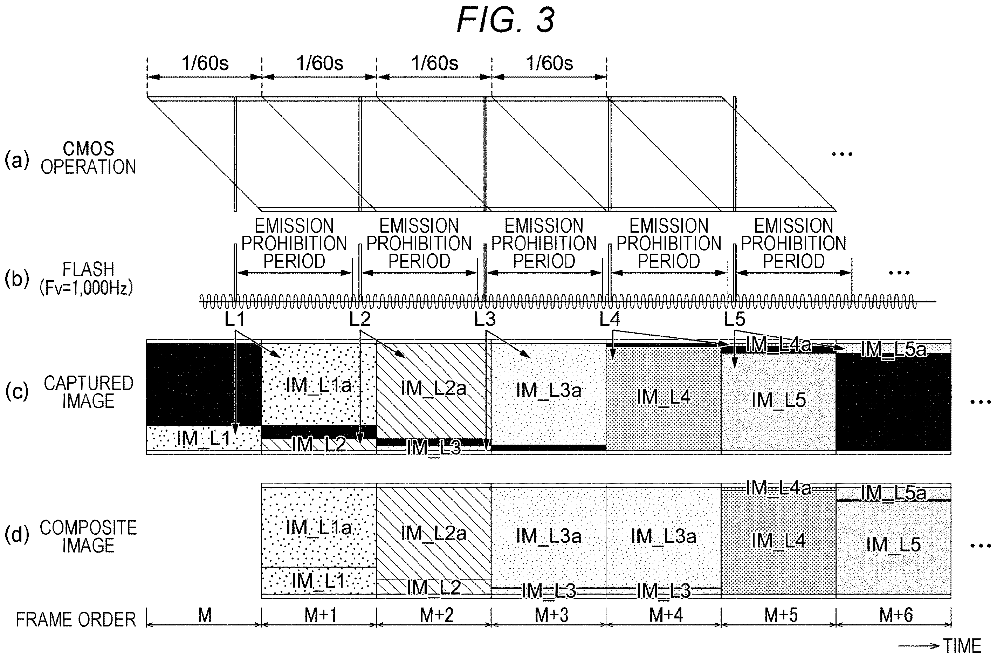

Next, with reference to the timing charts of Figs. 3 to 7, an example of the specific operation when a composite image is generated on the basis of the processes Pa and Pb in a case where the vocal cord vibration frequencies obtained from the microphone 40 are different in the electronic endoscope system 1 will be described. Note that each of Figs. 3 to 7 illustrates an example in which the emission prohibition period is set to approximately one frame period (1/60 second).

Each of Figs. 3 to 7 illustrates (a) the operation of the CMOS image sensor 14 (CMOS operation), (b) the emission timing of the flash, (c) the captured image obtained by the CMOS image sensor 14, and (d) the composite image generated by the synthesis unit 24 on a per-frame basis for the M-th frame, the (M + 1)-th frame, and the like. In (a) of Figs. 3 to 7, the emission period of the flash illustrated in (b) is indicated by vertical lines. In (b) of Figs. 3 to 7, the waveform of the vocal cord vibration is illustrated. The emission timings of the flashes L1, L2, L3, ... are adjusted according to a vocal cord vibration frequency Fv so as to satisfy the condition of the emission prohibition period.

### (I) Vocal cord vibration frequency Fv = 1000 Hz (Fig. 3)

In Fig. 3, since the flash L1 is emitted during the M-th frame period, a lower image IM_L1 captured with the flash L1 is obtained as the captured image of the M-th frame, and an upper image IM_L1a captured with the flash L1 is obtained as the captured image of the (M + 1)-th frame. Similarly, a lower image IM_L2 captured with the flash L2 is obtained as the captured image of the (M + 1)-th frame, and an upper image IM_L2a captured with the flash L2 is obtained as the captured image of the (M + 2)-th frame. A lower image IM_L3 captured with the flash L3 is obtained as the captured image of the (M + 2)-th frame, and an upper image IM_L3a captured with the flash L3 is obtained as the captured image of the (M + 3)-th frame.

In Fig. 3, since the flash L4 is emitted during the (M + 4)-th frame period, a lower image IM_L4 captured with the flash L4 is obtained as the captured image of the (M + 4)-th frame, and an upper image IM_L4a captured with the flash L4 is obtained as the captured image of the (M + 5)-th frame. Similarly, a lower image IM_L5 captured with the flash L5 is obtained as the captured image of the (M + 5)-th frame, and an upper image IM_L5a captured with the flash L5 is obtained as the captured image of the (M + 6)-th frame.

When the composite image of each frame from the (M + 1)-th to (M + 3)-th frame is created, a flash is emitted at any line of the CMOS image sensor 14 at the exposure start time of the frame immediately before each frame, and thus, the composite image is generated as follows according to the process Pa.

For example, when the composite image of the (M + 1)-th frame as the current frame is generated, the upper image corresponding to the lines above the flash start line of the M-th frame in the composite image is set as the upper image IM_L1a obtained from the corresponding lines of the (M + 1)-th frame (current frame), and the lower image corresponding to the lines below the flash end line of the M-th frame in the composite image is set as the lower image IM_L1 obtained from the corresponding lines of the M-th frame (frame that is one frame earlier). Note that, although not shown in Fig. 3, the boundary image between the flash start line and the flash end line in the composite image of the (M + 1)-th frame is an image obtained by adding the captured image obtained from the corresponding lines of the (M + 1)-th frame (current frame) and the captured image obtained from the corresponding lines of the frame that is one frame earlier.

The same applies to a case where a composite image is generated for each frame from the (M + 2)-th and (M + 3)-th frame as the current frame.

When the composite image of the (M + 4)-th frame as the current frame is created, the flash is not emitted at any line of the CMOS image sensor 14 at the exposure start time of the (M + 3)-th frame that is one frame earlier, and thus the composite image is generated as follows.

The upper image corresponding to lines above the flash start line of the (M + 2)-th frame that is two frames earlier in the composite image of the (M + 4)-th frame is set as a captured image IM_L3a obtained from the corresponding lines of the (M + 3)-th frame that is one frame earlier, and the lower image corresponding to lines below the flash end line of the (M + 2)-th frame that is two frames earlier in the composite image is set as a captured image IM_L3 obtained from the corresponding lines of the (M + 2)-th frame that is two frames earlier. Although not shown in Fig. 3, the boundary image between the flash start line and the flash end line in the composite image of the (M + 4)-th frame is an image obtained by adding the captured image obtained from the corresponding lines of the (M + 3)-th frame that is one frame earlier and the captured image obtained from the corresponding lines of the (M + 2)-th frame that is two frames earlier.

In short, when the composite image of the (M + 4)-th frame is created, the captured image IM_L4a is not obtained, and the composite image based on the captured images IM_L4 and IM_L4a cannot be created. Therefore, as illustrated in Fig. 3, the composite image of the (M + 3)-th frame is reproduced (that is, it is the same as the composite image of the (M + 3)-th frame).

For each frame from the (M + 5)-th and (M + 6)-th frame as the current frame, similarly to the case of creating the composite image of each frame from the (M + 1)-th to (M + 3)-th frame, the composite image is generated according to the process Pa.

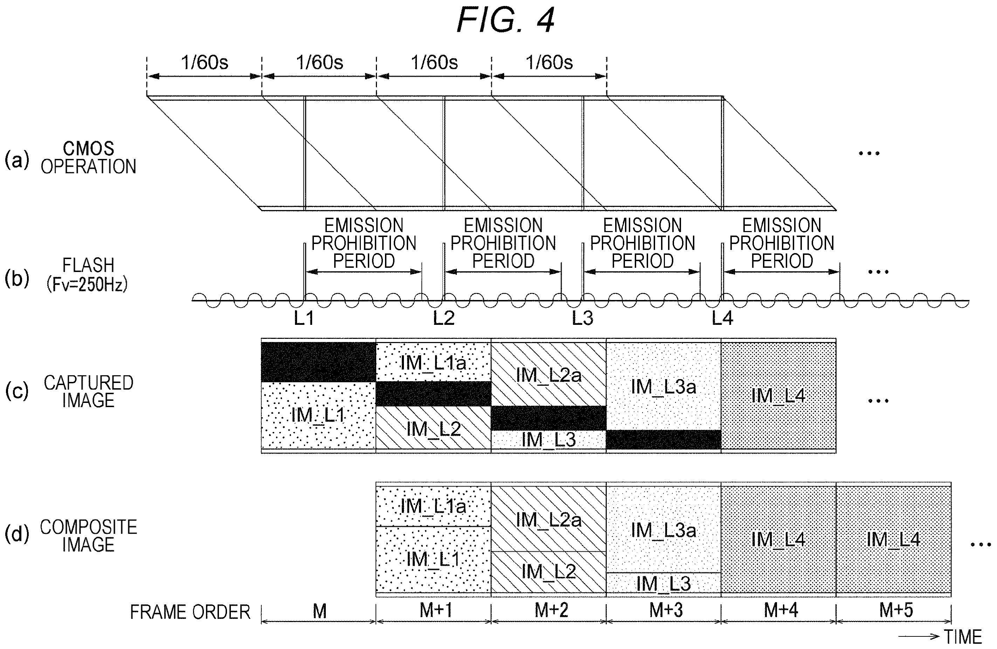

### (II) Vocal cord vibration frequency Fv = 250 Hz (Fig. 4)

As illustrated in Fig. 4(b), in a case where the vocal cord vibration frequency Fv is 250 Hz, a period from the end of the emission prohibition period after a certain flash until the emission of the next flash is longer than a period in a case where the vocal cord vibration frequency Fv is 1000 Hz.

In Fig. 4, since the flash L1 is emitted during the M-th frame period, the lower image IM_L1 captured with the flash L1 is obtained as the captured image of the M-th frame, and the upper image IM_L1a captured with the flash L1 is obtained as the captured image of the (M + 1)-th frame. Similarly, the lower image IM_L2 captured with the flash L2 is obtained as the captured image of the (M + 1)-th frame, and the upper image IM_L2a captured with the flash L2 is obtained as the captured image of the (M + 2)-th frame. The lower image IM_L3 captured with the flash L3 is obtained as the captured image of the (M + 2)-th frame, and the upper image IM_L3a captured with the flash L3 is obtained as the captured image of the (M + 3)-th frame.

In Fig. 4, the flash L4 is emitted during the (M + 4)-th frame period, and the image IM_L4 of the entire effective pixel region, which is captured with the flash L4, is obtained as the captured image of the (M + 4)-th frame.

When the composite image of each frame from the (M + 1)-th to (M + 3)-th frame as the current frame is created, a flash is emitted at any line of the CMOS image sensor 14 at the exposure start time of the frame immediately before each frame, and thus, the composite image is generated according to the process Pa. The generating of this composite image is the same as the case of creating a composite image of each frame from the (M + 1)-th to (M + 3)-th frame in the case of vocal cord vibration frequency Fv = 1000 Hz in (I).

The composite image is also generated for the (M + 4)-th frame as the current frame in accordance with the process Pa. However, since the flash is emitted in the inactive pixel region of the CMOS image sensor 14 at the exposure start time of the (M + 3)-th frame that is one frame earlier, substantial synthesis is not performed, and the composite image of the (M + 4)-th frame is the captured image IM_L4.

Since the (M + 5)-th frame as the current frame corresponds to a case where the flash is not emitted at any line of the CMOS image sensor 14 at the exposure start time of the (M + 4)-th frame that is one frame earlier, the composite image is generated according to the process Pb. As a result, the composite image of the (M + 5)-th frame is identical to the composite image of the (M + 4)-th frame.

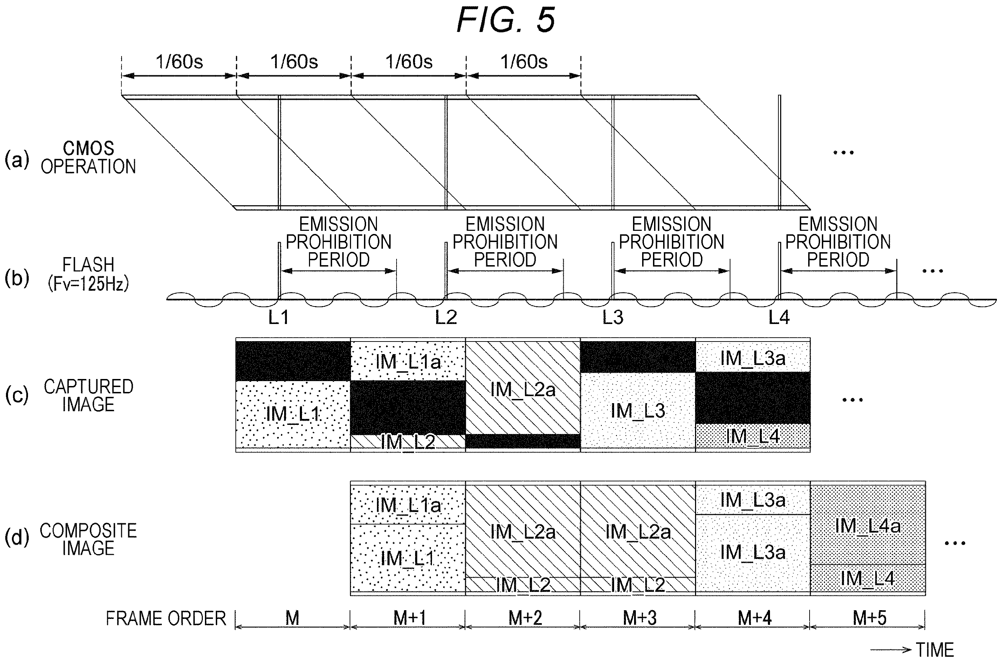

### (III) Vocal cord vibration frequency Fv = 125 Hz (Fig. 5)

As illustrated in Fig. 5(b), in a case where the vocal cord vibration frequency Fv is 125 Hz, a period from the end of the emission prohibition period after a certain flash until the emission of the next flash is even longer than a period in a case where the vocal cord vibration frequency Fv is 250 Hz.

In Fig. 5, since the flash L1 is emitted during the M-th frame period, the lower image IM_L1 captured with the flash L1 is obtained as the captured image of the M-th frame, and the upper image IM_L1a captured with the flash L1 is obtained as the captured image of the (M + 1)-th frame. Similarly, the lower image IM_L2 captured with the flash L2 is obtained as the captured image of the (M + 1)-th frame, and the upper image IM_L2a captured with the flash L2 is obtained as the captured image of the (M + 2)-th frame. The lower image IM_L3 captured with the flash L3 is obtained as the captured image of the (M + 3)-th frame, and the upper image IM_L3a captured with the flash L3 is obtained as the captured image of the (M + 4)-th frame. As the captured image of the (M + 4)-th frame, the lower image IM_L4 captured with the flash L4 is obtained.

When the composite image of each frame from the (M + 1)-th and (M + 2)-th frame as the current frame is created, a flash is emitted at any line of the CMOS image sensor 14 at the exposure start time of the frame immediately before each frame, and thus, the composite image is generated according to the process Pa. The generating of this composite image is the same as the case of creating a composite image of each frame from the (M + 1)-th to (M + 3)-th frame in the case of vocal cord vibration frequency Fv = 1000 Hz in (I).

Since the (M + 3)-th frame as the current frame corresponds to a case where the flash is not emitted at any line of the CMOS image sensor 14 at the exposure start time of the (M + 2)-th frame that is one frame earlier, the composite image is generated according to the process Pb. As a result, the composite image of the (M + 3)-th frame is identical to the composite image of the (M + 2)-th frame.

When the composite image of each frame from the (M + 4)-th and (M + 5)-th frame as the current frame is created, a flash is emitted at any line of the CMOS image sensor 14 at the exposure start time of the frame immediately before each frame, and thus, the composite image is generated according to the process Pa. That is, the composite image of each frame from the (M + 4)-th and (M + 5)-th frame is created in a similar manner to the case of creating the composite image of each frame from the (M + 1)-th and (M + 2)-th frame.

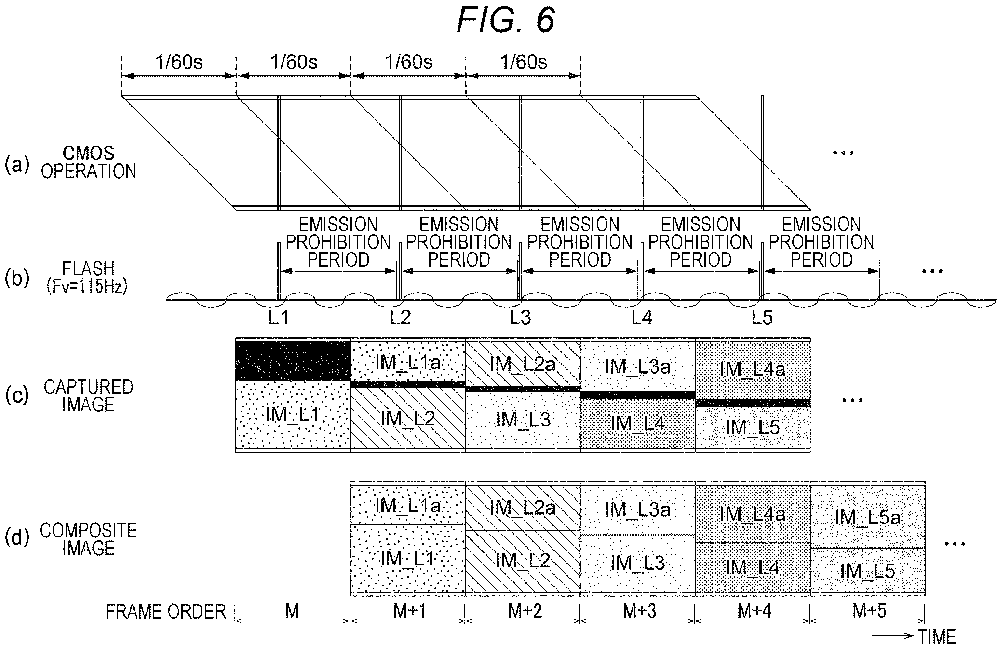

### (IV) Vocal cord vibration frequency Fv = 115 Hz (Fig. 6)

As illustrated in Fig. 6(b), in a case where the vocal cord vibration frequency Fv is 115 Hz, a period from the end of the emission prohibition period after a certain flash until the emission of the next flash is significantly short.

In Fig. 6, since the flash L1 is emitted during the M-th frame period, the lower image IM_L1 captured with the flash L1 is obtained as the captured image of the M-th frame, and the upper image IM_L1a captured with the flash L1 is obtained as the captured image of the (M + 1)-th frame. Similarly, the lower image IM_L2 captured with the flash L2 is obtained as the captured image of the (M + 1)-th frame, and the upper image IM_L2a captured with the flash L2 is obtained as the captured image of the (M + 2)-th frame. The lower image IM_L3 captured with the flash L3 is obtained as the captured image of the (M + 2)-th frame, and the upper image IM_L3a captured with the flash L3 is obtained as the captured image of the (M + 3)-th frame. The lower image IM_L4 captured with the flash L4 is obtained as the captured image of the (M + 3)-th frame, and the upper image IM_L4a captured with the flash L4 is obtained as the captured image of the (M + 4)-th frame. As the captured image of the (M + 4)-th frame, the lower image IM_L5 captured with the flash L5 is obtained.

When the composite image of each frame from the (M + 1)-th to (M + 5)-th frame as the current frame is created, a flash is emitted at any line of the CMOS image sensor 14 at the exposure start time of the frame immediately before each frame, and thus, the composite image is generated according to the process Pa. The generating of this composite image is the same as the case of creating a composite image of each frame from the (M + 1)-th to (M + 3)-th frame in the case of vocal cord vibration frequency Fv = 1000 Hz in (I).

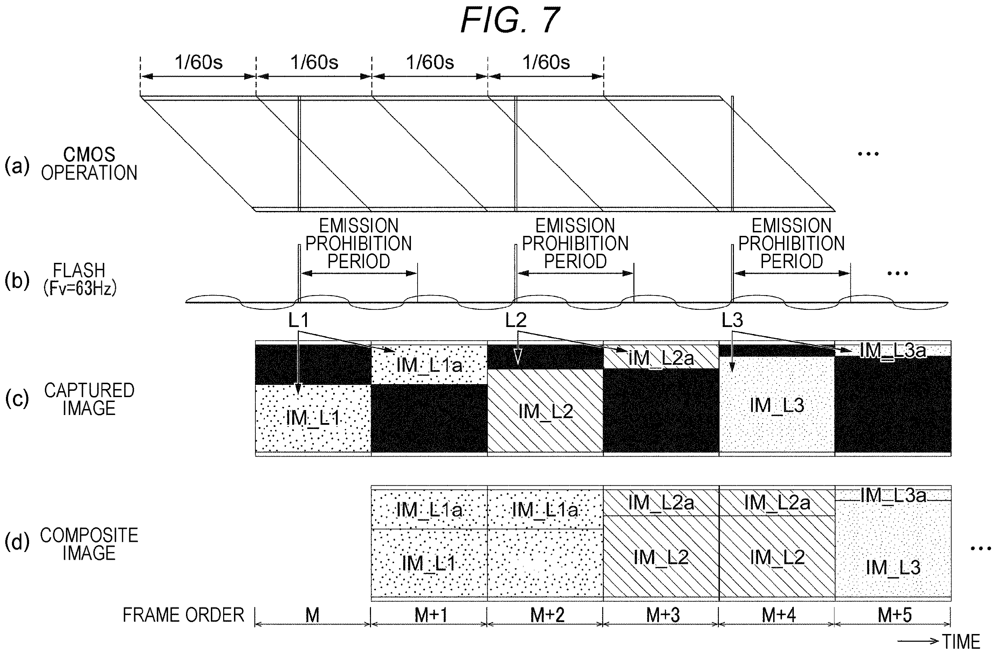

### (V) Vocal cord vibration frequency Fv = 63 Hz (Fig. 7)

As illustrated in Fig. 7(b), in a case where the vocal cord vibration frequency Fv is 63 Hz, a period from the end of the emission prohibition period after a certain flash until the emission of the next flash is significantly long.

In Fig. 7, since the flash L1 is emitted during the M-th frame period, the lower image IM_L1 captured with the flash L1 is obtained as the captured image of the M-th frame, and the upper image IM_L1a captured with the flash L1 is obtained as the captured image of the (M + 1)-th frame. Similarly, the lower image IM_L2 captured with the flash L2 is obtained as the captured image of the (M + 2)-th frame, and the upper image IM_L2a captured with the flash L2 is obtained as the captured image of the (M + 3)-th frame. The lower image IM_L3 captured with the flash L3 is obtained as the captured image of the (M + 4)-th frame, and the upper image IM_L3a captured with the flash L3 is obtained as the captured image of the (M + 5)-th frame.

When the composite image of the (M + 1)-th frame as the current frame is created, a flash is emitted at any line of the CMOS image sensor 14 at the exposure start time of the frame immediately before the (M + 1)-th frame (the M-th frame), and thus, the composite image is generated according to the process Pa.

When the composite image of the (M + 2)-th frame as the current frame is created, the flash is not emitted at any line of the CMOS image sensor 14 at the exposure start time of the frame immediately before the (M + 2)-th frame (the (M + 1)-th frame), and thus, the composite image is generated according to the process Pb.

When the composite image of the (M + 3)-th frame as the current frame is created, a flash is emitted at any line of the CMOS image sensor 14 at the exposure start time of the frame immediately before the (M + 3)-th frame (the (M + 2)-th frame), and thus, the composite image is generated according to the process Pa.

When the composite image of the (M + 4)-th frame as the current frame is created, the flash is not emitted at any line of the CMOS image sensor 14 at the exposure start time of the frame immediately before the (M + 4)-th frame (the (M + 3)-th frame), and thus, the composite image is generated according to the process Pb.

When the composite image of the (M + 5)-th frame as the current frame is created, a flash is emitted at any line of the CMOS image sensor 14 at the exposure start time of the frame immediately before the (M + 5)-th frame (the (M + 4)-th frame), and thus, the composite image is generated according to the process Pa.

As a result, the composite image of each frame is generated as illustrated in Fig. 7.

As specifically described above with reference to Figs. 3 to 7, in the electronic endoscope system 1, the emission timing of the flash is determined such that the emission interval of consecutive flashes becomes longer than a predetermined emission prohibition period and synchronized with the vocal cord vibration frequency.

At this time, in a case where the flash is not emitted at any line of the CMOS image sensor 14 at the exposure start time of the CMOS image sensor 14 operating by the rolling shutter method, the composite images of two consecutive frames become the same, and the frame rate decreases. For example, as illustrated in Fig. 6, in a case where the vocal cord vibration frequency Fv is 115 Hz, the flash is emitted at any line of the CMOS image sensor 14 at the exposure start time for any frame. Therefore, the composite images of the frames are not the same, and the frame rate is high (about 60 fps). On the other hand, as illustrated in Fig. 7, in a case where the vocal cord vibration frequency Fv is 63 Hz, a situation where the flash is not emitted at any line of the CMOS image sensor 14 at the exposure start time occurs every other frame. Therefore, the composite images are the same for every two consecutive frames, and the frame rate is low (about 30 fps).

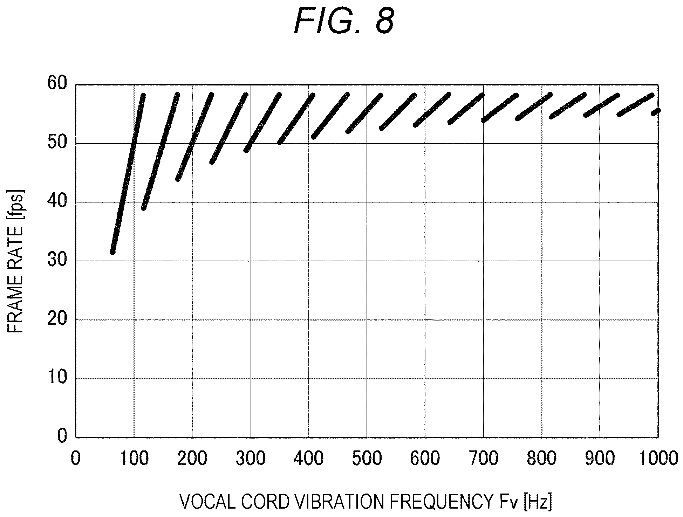

Fig. 8 illustrates the relationship between the vocal cord vibration frequency Fv and the frame rate of the composite image.

In the electronic endoscope system 1 of the present embodiment, the frame rate varies discontinuously on the basis of the vocal cord vibration frequency Fv and the emission timing of the flash set according to a predetermined emission prohibition period. This is because a period from the end of the emission prohibition period after a certain flash until the emission of the next flash changes discontinuously according to the vocal cord vibration frequency.

Referring to Fig. 8, it can be seen that the frame rate of at least 30 fps is secured for any vocal cord vibration frequency, and as the vocal cord vibration frequency increases, the frame rate approaches 60 fps, and a high frame rate is obtained. When the total length of the flash exposure period and the flash prohibition period matches the cycle corresponding to vocal cord vibration frequency Fv, the frame rate increases. Therefore, the frame rate periodically increases with respect to the vocal cord vibration frequency Fv as illustrated in Fig. 8.

Note that Fig. 8 illustrates a result obtained in a case where the emission prohibition period is set to approximately one frame period (1/60 second) as illustrated in Figs. 3 to 7, and a result different from that in Fig. 8 is obtained in a case where the emission prohibition period is further lengthened.

Next, preferable post-processing performed on the composite image obtained by the processes Pa and Pb will be described.

As described above, in the process Pa, the boundary image between the flash start line and the flash end line in the composite image of the current frame is an image obtained by adding the captured image obtained from the corresponding lines of the current frame and the captured image obtained from the corresponding lines of the frame that is one frame earlier. On the other hand, in the process Pb, the boundary image between the flash start line and the flash end line in the composite image of the current frame is an image obtained by adding the captured image obtained from the corresponding lines of the frame that is one frame earlier and the captured image obtained from the corresponding lines of the frame that is two frames earlier.

The boundary image in the composite image is a region sandwiched between the upper image and the lower image in the composite image. As illustrated in Fig. 2, this boundary image is an image during a period when the flash exposure period (Ts to Te) of the flash L and the signal reading period T_{RO} at least partially overlap and exposure by the flash L is not sufficiently performed. Therefore, when no processing is performed on this boundary image, as illustrated in (d) of Figs. 3 to 7, the boundary image becomes conspicuous as a horizontal black line in the composite image, and is visually recognized by the observer as a black line moving in the vertical direction.

Thus, it is preferable to perform image processing to described below on the boundary image and the image corresponding to the lines in the vicinity of the boundary image as post-processing on the composite image such that the horizontal black line of the boundary image is not conspicuous in the composite image.

In the embodiment, the post-processing on the composite image corresponds to one of the following three processes Pc1 to Pc3, or a combination of two or more processes.

### [Process Pc1] Digital gain application processing

The digital gain application processing is processing of amplifying the pixel value of each pixel in each line of the boundary image. By applying the digital gain, the luminance of the boundary image obtained in a state where the exposure by the flash is not sufficient is corrected.

### [Process Pc2] Spatial filter processing

The spatial filter processing is processing of applying a spatial filter for smoothing (blurring) the image to the pixel values of the pixels of the boundary image included in the composite image of the current frame, or to the pixel values of the pixels of the boundary image and a predetermined number of lines in the vicinity of the boundary image.

### [Process Pc3] Frame interpolation processing

The frame interpolation processing is processing of performing interpolation between the current frame and the past frame that is two frames earlier or three frames earlier on the boundary image included in the current frame or the boundary image and a predetermined number of lines in the vicinity of the boundary image.

Although only the process Pc1 may be used, it is preferable to combine the process Pc1 and the process Pc2 or combine the process Pc1 and the process Pc3 in order to make the horizontal black line of the boundary image more effectively inconspicuous in the composite image.

### (i) First aspect of post-processing on composite image

A first aspect of the post-processing on the composite image is processing of combining the processing Pc1 and the processing Pc2.

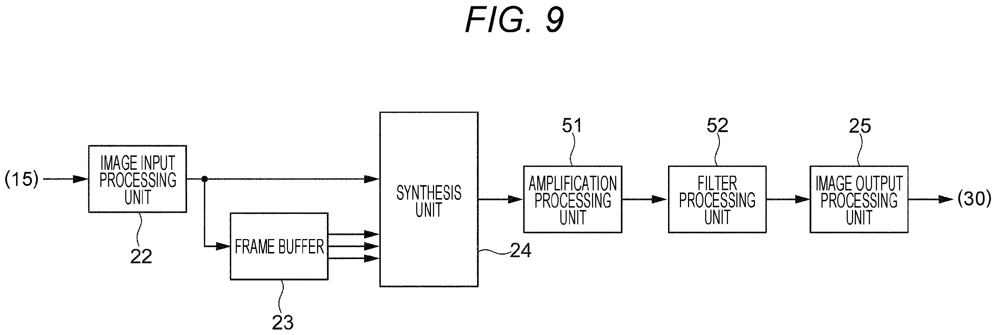

Fig. 9 illustrates a system configuration in a case where the first aspect is implemented. As illustrated in Fig. 9, in the first aspect, the processor 20 illustrated in Fig. 1 further includes an amplification processing unit 51 and a filter processing unit 52 between the synthesis unit 24 and the image output processing unit 25.

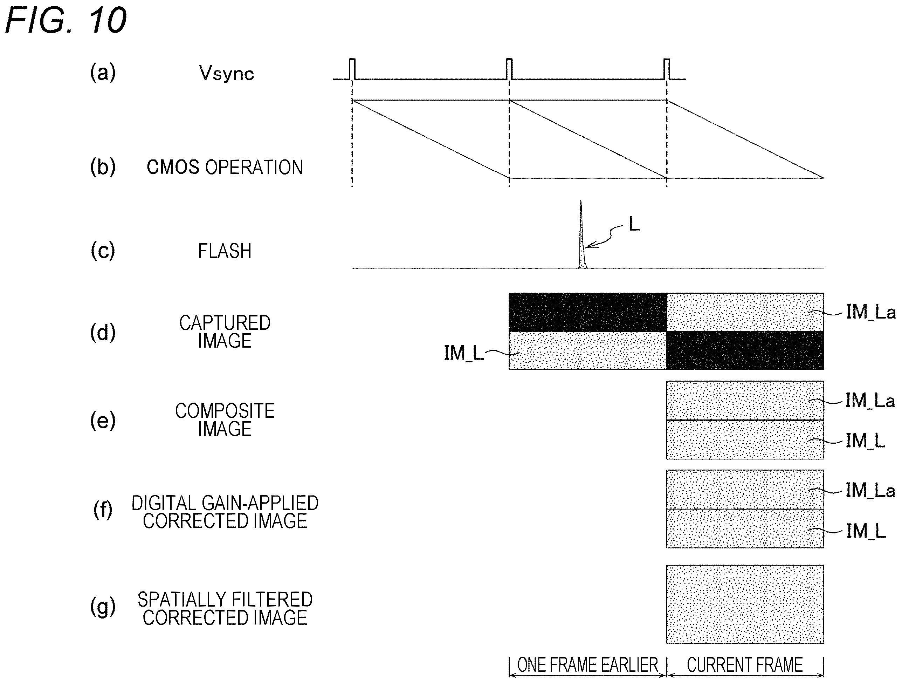

Fig. 10 is a diagram illustrating image processing in a case where the first aspect regarding post-processing on a composite image is applied. In Fig. 10, (a) illustrates a vertical synchronization signal Vsync of the CMOS image sensor 14, (b) illustrates the operation (CMOS operation) of the CMOS image sensor 14, (c) illustrates a flash L, (d) illustrates a captured image acquired by the CMOS image sensor 14, (e) illustrates a composite image, (f) illustrates a digital gain-applied corrected image, and (g) illustrates a spatially filtered corrected image.

Referring to Fig. 10, as illustrated in Figs. 10(a) and 10(b), the CMOS image sensor 14 starts exposure using the rolling shutter method for each frame in synchronization with the vertical synchronization signal Vsync. As illustrated in Fig. 10(d), with the current frame as a reference, the lower image IM_L is obtained as the captured image that is one frame earlier from the flash L emitted one frame earlier, and the upper image IM_La is obtained as the captured image of the current frame. As illustrated in Fig. 10(e), the composite image of the current frame is an image obtained by synthesizing the lower image IM_L and the upper image IM_La, and in this composite image, a horizontal black line is conspicuous in the boundary image.

The amplification processing unit 51 illustrated in Fig. 9 generates a digital gain-applied corrected image (Fig. 10(f)) by amplifying the pixel value (digital value) of each pixel of each line of the boundary image in the composite image of the current frame generated by the synthesis unit 24. Thus, the luminance of each pixel of each line of the boundary image increases, and the horizontal black line becomes less conspicuous in the boundary image.

The filter processing unit 52 illustrated in Fig. 9 applies spatial filter to the pixel values of the pixels included in a predetermined number of upper and lower lines from an adjacent position where the boundary image and the upper image IM_La are adjacent to each other and a predetermined number of upper and lower lines from an adjacent position where the boundary image and the lower image IM_L are adjacent to each other for the composite image of the current frame, and generates a spatially filtered corrected image (Fig. 10(g)). Thus, smoothing is performed on the boundary image and the image in the vicinity of the boundary image, and the horizontal black line becomes more inconspicuous in the boundary image.

The spatial filter applied here may be any known spatial filter as long as the spatial filter smooths the boundary image and the upper image IM_La and the boundary image and the lower image IM_L, and examples thereof include a Gaussian filter and an averaging filter.

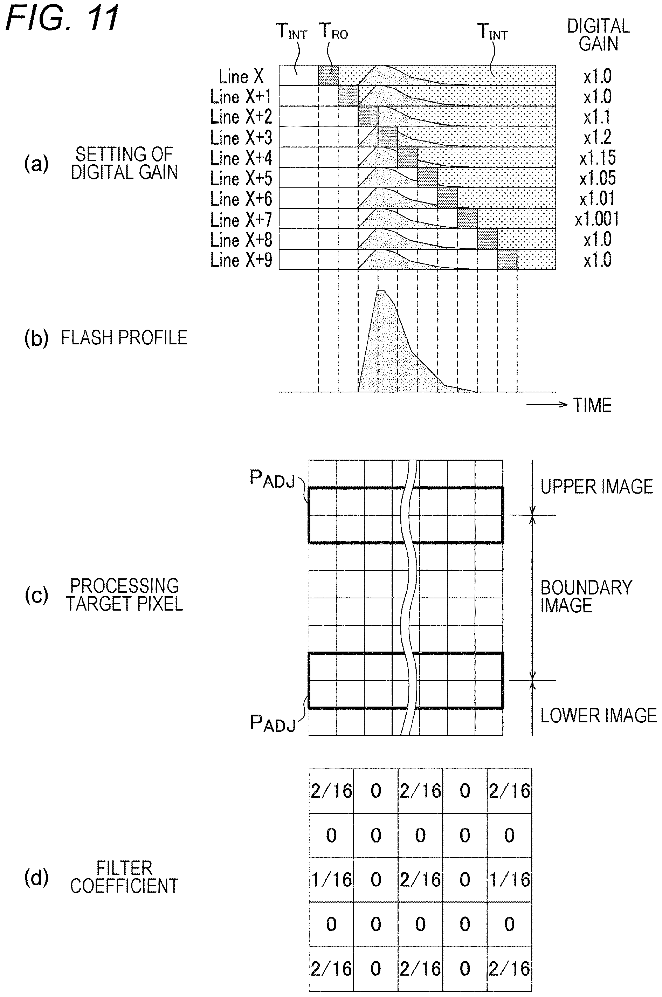

Next, a specific setting example of the spatial filter in the first aspect for the post-processing on the composite image will be described with reference to Figs. 11 to 13.

In each of Figs. 11 to 13, (a) illustrates the setting of the digital gain for each pixel of each line of the boundary image, (b) illustrates the flash profile, (c) illustrates processing target pixels for the spatial filter, and (d) illustrates filter coefficients (kernel) of the spatial filter. Similarly to Fig. 2(c), (a) of each of Figs. 11 to 13 illustrates the charge accumulation period T_{IN}T and the signal reading period T_{RO} for a plurality of lines (Line X to Line X+9) in the vicinity of the flash start line and the flash end line, and also illustrates the flash profile illustrated in (b) of each of Figs. 11 to 13 compressed in the vertical direction.

Note that the flash profile is an emission intensity profile corresponding to the elapse of time during the flash, in other words, the flash profile means the emission characteristics of the flash when the horizontal axis represents time and the vertical axis represents the emission intensity of the flash.

In Fig. 2(b), an ideal pulsed flash profile is illustrated for convenience of description, but the actual flash profile may vary depending on the magnitude of the emission intensity. For example, (b) of Figs. 11 to 13 illustrates a flash caused by the same LED, and illustrates a flash profile when the emission intensity is decreased (that is, the LED current is decreased) in the order of Fig. 11(b), Fig. 12(b), and Fig. 13(b). As described above, when the emission intensity of the LED is varied, a flash profile (in particular, at the time of rising and at the time of falling) of the LED varies according to a change in characteristics of a drive circuit, a power supply circuit, and the like in the light source device 28.

Furthermore, referring to (a) of Figs. 11 to 13, the exposure amount of each line of the boundary image changes according to the degree of overlap between the flash profile and the signal reading period T_{RO} when electric charge cannot be accumulated.

Therefore, preferably, the amplification processing unit 51 makes the gain applied to the pixel of each line of the boundary image in the composite image of the current frame larger than the gain applied to the pixel of each line in the images other than the boundary image according to the flash profile and the signal reading period of the CMOS image sensor 14.

For example, in the digital gain setting example of Fig. 11(a), since the flash profile and the signal reading period T_{RO} hardly overlap in Line X and Line X+1, the digital gain is set to "1.0", but the signal reading period T_{RO} greatly overlaps the flash profile in Line X+3. That is, since the portion of the peak of the flash corresponds to the signal reading period T_{RO} and is not exposed in any of the preceding and subsequent frames, the digital gain is set to "1.2". In Line X+2 to Line X+7, since there is a portion that is not exposed in any of the preceding and subsequent frames in the flash profile, when the boundary image is not amplified, the horizontal black line becomes easily conspicuous in the boundary image. Thus, a decrease in the exposure amount is compensated by setting a digital gain (that is, a gain that substantially amplifies the pixel value) larger than one for these lines. At this time, as illustrated in Fig. 11(a), since a gain is set larger as the charge accumulation amount (or the integrated emission intensity) of each line of the boundary image is smaller, the luminance of each pixel can be effectively increased.

(c) of Figs. 11 to 13 illustrates an example of a processing target pixel group P_{ADJ} of the filter processing.

The processing target pixel group P_{ADJ} is a pixel group included in a predetermined number of upper and lower lines from an adjacent position where the boundary image and the upper image are adjacent to each other and a predetermined number of upper and lower lines from an adjacent position where the boundary image and the lower image are adjacent to each other. The filter processing unit 52 performs filter processing on each pixel included in the processing target pixel group P_{ADJ} using the filter coefficients illustrated in (d) of Figs. 11 to 13.

The 5×5 filter coefficients illustrated in (d) of Figs. 11 to 13 are filter coefficients in a case where a color filter of the Bayer arrangement is applied to each pixel, and are substantially the 3×3 filter coefficients.

Preferably, the filter processing unit 52 sets the filter coefficient of the spatial filter applied to the pixels included in each line of a predetermined number of lines according to the flash profile.

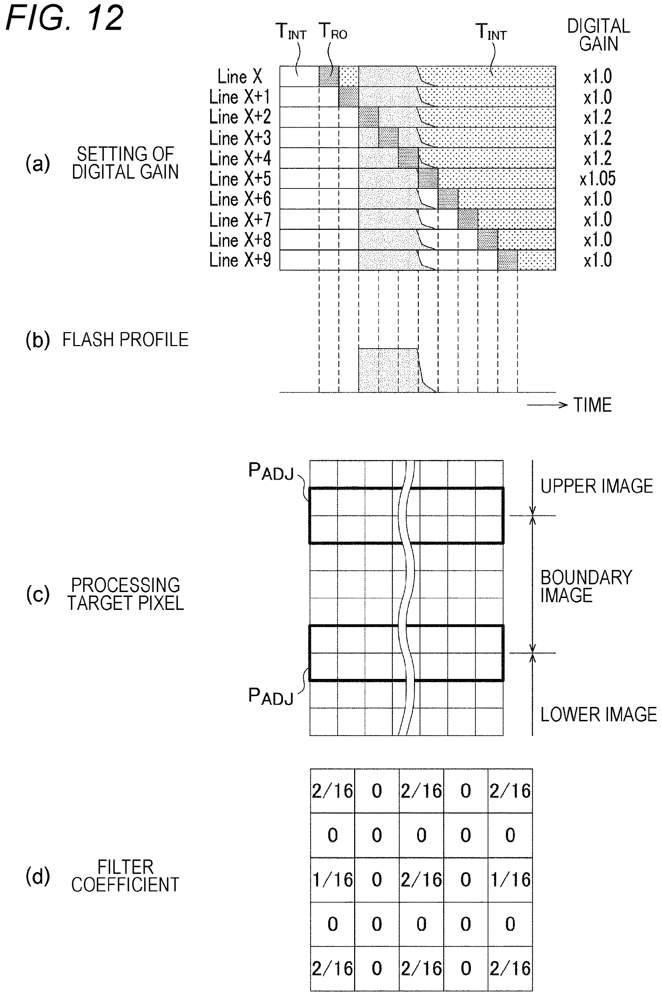

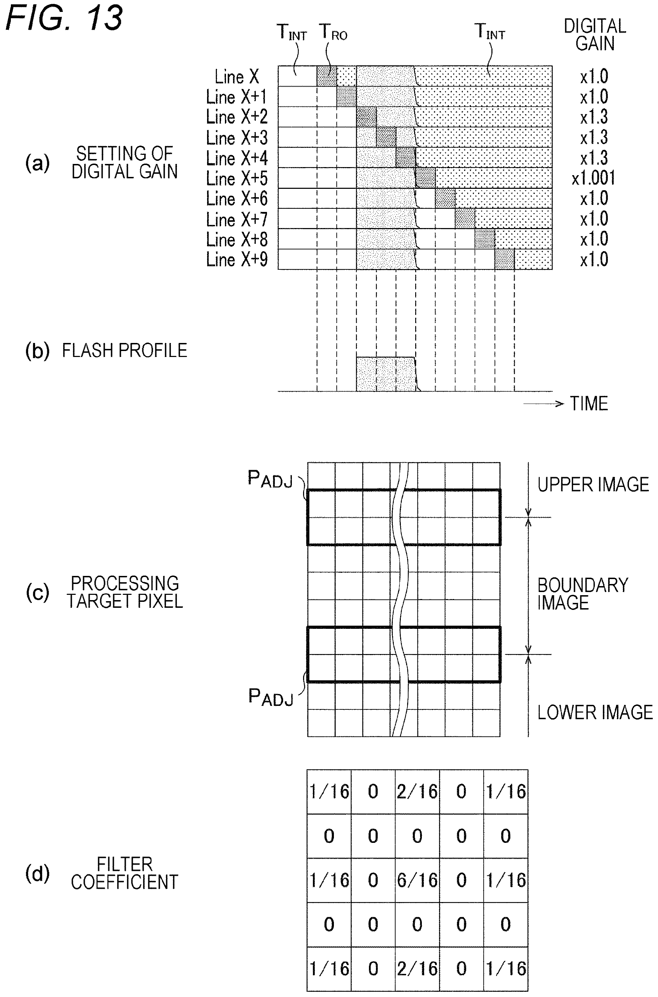

For example, in the filter coefficients illustrated in Fig. 13(d), the weight of the target pixel is large and the weight of the pixel in the vertical direction is low as compared with the filter coefficients illustrated in Figs. 11(d) and 12(d). By optimizing the filter coefficients according to the flash profile, an appropriate smoothing processing can be performed on the flash profile.

In one embodiment, the filter processing unit 52 applies a spatial filter to the pixel values of the pixels included in a predetermined number of upper and lower lines from the central position in the line direction of the boundary image for the composite image of the current frame. That is, unlike the processing target pixel group P_{ADJ} illustrated in (c) of Figs. 11 to 13, the processing target pixel group may be a pixel group included in a predetermined number of upper and lower lines from the central position in the line direction of the boundary image. In this case, smoothing is performed on the boundary image and the images in the vicinity of the boundary image, and the horizontal black line can be made more inconspicuous in the boundary image.

### (ii) Second aspect of post-processing on composite image

A second aspect of the post-processing on the composite image is processing of combining the processing Pc1 and the processing Pc3.

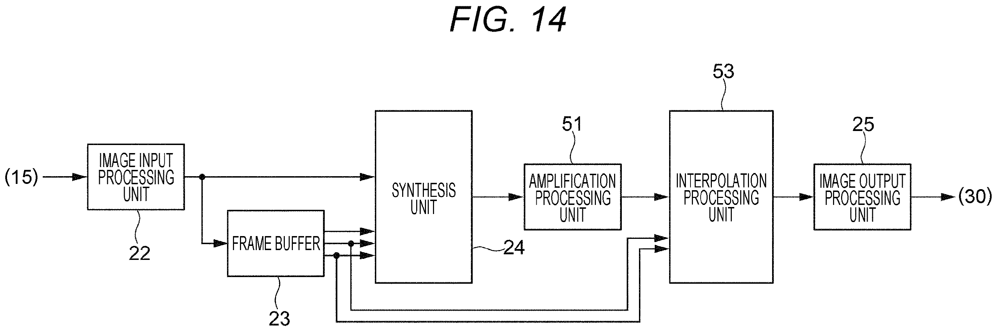

Fig. 14 illustrates a system configuration in a case where the second aspect is implemented. As illustrated in Fig. 14, in the second aspect, the processor 20 illustrated in Fig. 1 further includes an amplification processing unit 51 and an interpolation processing unit 53 between the synthesis unit 24 and the image output processing unit 25.

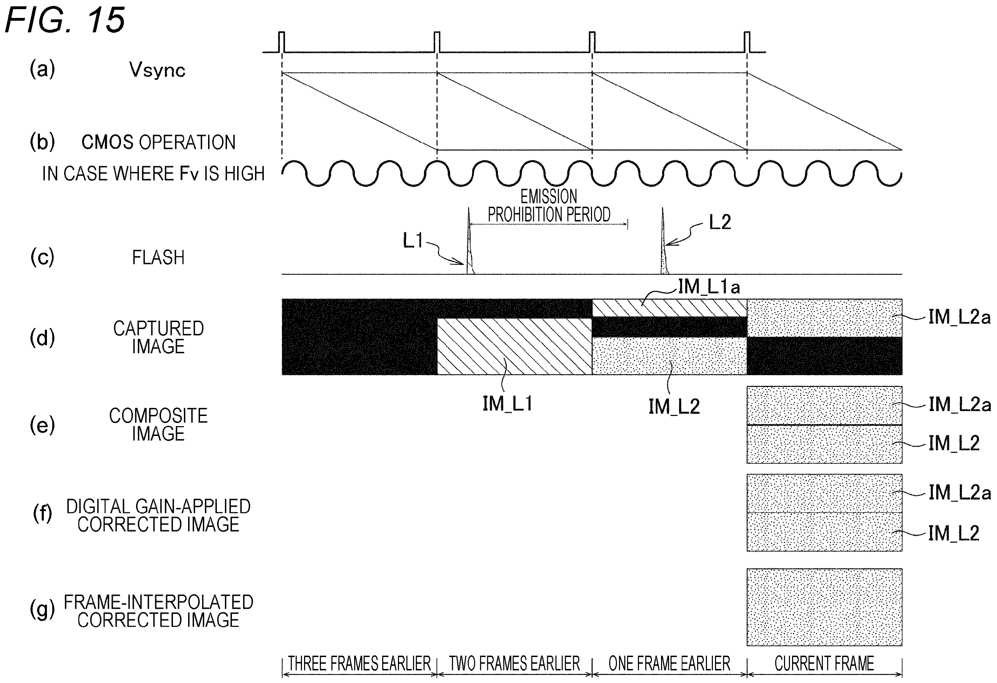

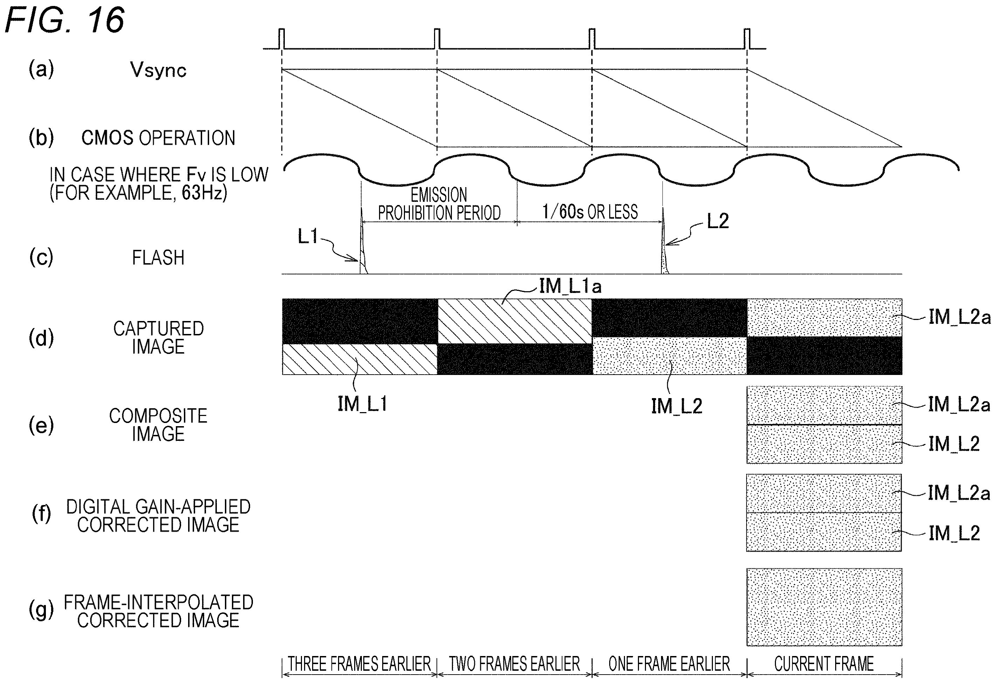

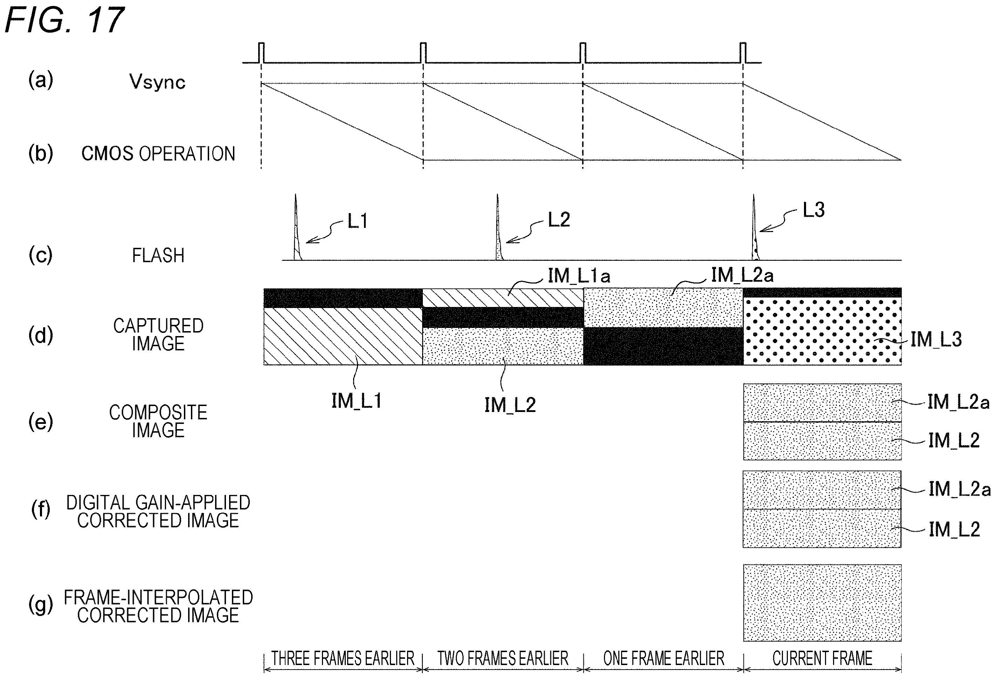

Figs. 15 and 17 are diagrams illustrating image processing in a case where the second aspect regarding the post-processing on the composite image is applied. In each of Figs. 15 to 17, (a) illustrates a vertical synchronization signal Vsync of the CMOS image sensor 14, (b) illustrates the operation (CMOS operation) of the CMOS image sensor 14, (c) illustrates a flash L, (d) illustrates a captured image acquired by the CMOS image sensor 14, (e) illustrates a composite image, (f) illustrates a digital gain-applied corrected image, and (g) illustrates a frame-interpolated corrected image. Note that Figs. 15(c) and 16(c) illustrate the waveform of vocal cord vibration that is the basis of the emission timing of the flash. Fig. 15(c) illustrates the flash timing in a case where the vocal cord vibration frequency Fv is relatively high, and Fig. 16(c) illustrates the flash timing in a case where the vocal cord vibration frequency Fv is low (for example, Fv = 63 Hz).

Referring to Fig. 15, as illustrated in Figs. 15(a) and 15(b), the CMOS image sensor 14 starts exposure using the rolling shutter method for each frame in synchronization with the vertical synchronization signal Vsync. As illustrated in Fig. 15(d), with the current frame as a reference, the lower image IM_L1 is obtained as the captured image that is two frames earlier from the flash L1, the upper image IM_L1a is obtained as the captured image that is one frame earlier from the flash L1, the lower image IM_L2 is obtained as the captured image that is one frame earlier from the flash L2, and the upper image IM_L2a is obtained as the captured image of the current frame from the flash L2. As illustrated in Fig. 15(e), the composite image of the current frame is an image obtained by synthesizing the lower image IM_L2 and the upper image IM_L2a, and in this composite image, a horizontal black line is conspicuous in the boundary image.

Referring to Fig. 16, as illustrated in Fig. 15, the CMOS image sensor 14 starts exposure using the rolling shutter method for each frame in synchronization with the vertical synchronization signal Vsync. In Fig. 16, the vocal cord vibration frequency Fv is lower than that in Fig. 15, and the emission timings of the flashes L1 and L2 are different from those in Fig. 15. Therefore, as illustrated in Fig. 16(d), with the current frame as a reference, the lower image IM_L1 is obtained as the captured image that is three frames earlier from the flash L1, the upper image IM_L1a is obtained as the captured image that is two frames earlier from the flash L1, the lower image IM_L2 is obtained as the captured image that is one frame earlier from the flash L2, and the upper image IM_L2a is obtained as the captured image of the current frame from the flash L2. As illustrated in Fig. 16(e), the composite image of the current frame is an image obtained by synthesizing the lower image IM_L2 and the upper image IM_L2a, and in this composite image, a horizontal black line is conspicuous in the boundary image as illustrated in Fig. 15.

Unlike Figs. 15 and 16, Fig. 17 illustrates image processing in a case where a flash is not emitted at any line of the CMOS image sensor 14 at the exposure start time of the frame one frame before the current frame. In this example, as illustrated in Fig. 17(d), with the current frame as a reference, the lower image IM_L1 is obtained as the captured image that is three frames earlier from the flash L1, the upper image IM_L1a is obtained as the captured image that is two frames earlier from the flash L1, the lower image IM_L2 is obtained as the captured image that is two frames earlier from the flash L2, and the upper image IM_L2a is obtained as the captured image that is one frame earlier from the flash L2. As illustrated in Fig. 17(e), the composite image of the current frame is an image obtained by synthesizing the lower image IM_L2 and the upper image IM_L2a, and in this composite image, a horizontal black line is conspicuous in the boundary image as illustrated in Figs. 15 and 16.

The amplification processing unit 51 illustrated in Fig. 14 generates a digital gain-applied corrected image (Figs. 15(f) to 17(f)) by amplifying the pixel value (digital value) of each pixel of each line of the boundary image in the composite image of the current frame generated by the synthesis unit 24. Thus, the luminance of each pixel of each line of the boundary image increases, and the horizontal black line becomes less conspicuous in the boundary image. This point is the same as the first aspect in Fig. 9.

The interpolation processing unit 53 illustrated in Fig. 14 sets, as the target line, each of the lines of the boundary image in the composite image of the current frame or each of a plurality of lines including a predetermined number of lines above the adjacent position where the boundary image and the upper image are adjacent to each other and each of a predetermined number of lines below the adjacent position where the boundary image and the lower image are adjacent to each other, as well as each of the lines of the boundary image, and performs interpolation processing between the current frame and the past frame two or three frames before the current frame. Specifically, the interpolation processing unit 53 reads captured images that are two frames earlier and three frames earlier, which are stored in the frame buffer 23, and performs frame interpolation processing as follows.

As illustrated in Figs. 15 and 16, in a case where a flash is emitted at any line of the CMOS image sensor 14 at the exposure start time that is one frame before the current frame, the interpolation processing unit 53 calculates the pixel values of the pixels included in each target line in the composite image of the current frame by performing weighted averaging on the pixel values of the corresponding pixels in the composite image of the current frame and the pixel values of the corresponding pixels in the captured image two frames earlier (the lower image IM_L1 in Fig. 15 and the upper image IM_L1a in Fig. 16).

As illustrated in Fig. 17, in a case where the flash is not emitted at any line of the CMOS image sensor 14 at the exposure start time that is one frame before the current frame, the interpolation processing unit 53 calculates the pixel values of the pixels included in each target line in the composite image of the current frame by performing weighted averaging on the pixel values of the corresponding pixels in the composite image of the current frame and the pixel values of the corresponding pixels in the captured image three frames earlier. In the example illustrated in Fig. 17, the lower image IM_L1, which is the captured image that is three frames earlier is the basis of the frame interpolation in the composite image of the current frame.

As described above, by performing the frame interpolation processing, it is possible to make the horizontal black line less conspicuous in the boundary image and to prevent a sense of discomfort when viewed as a moving image.

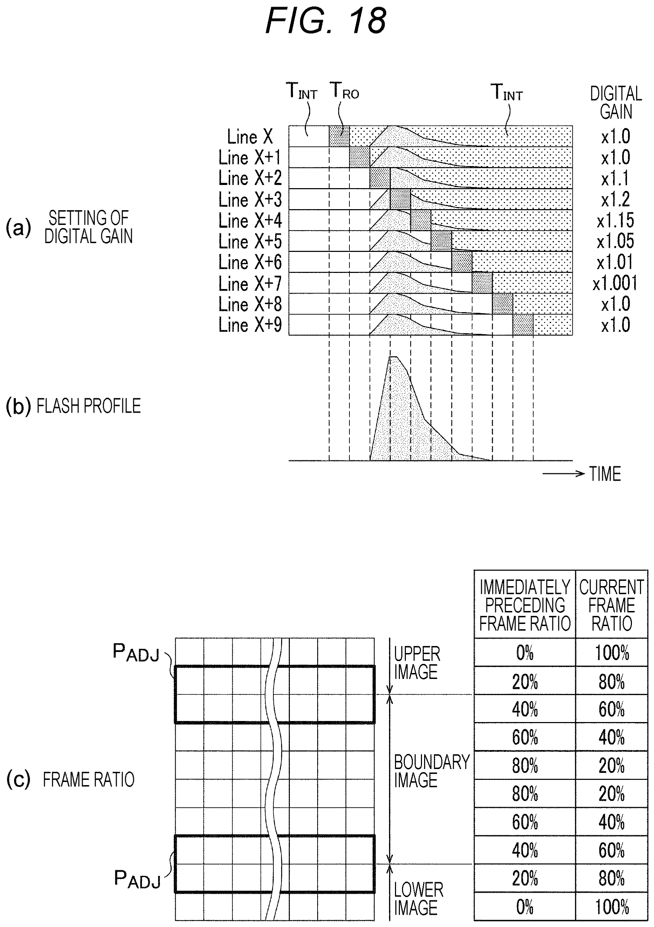

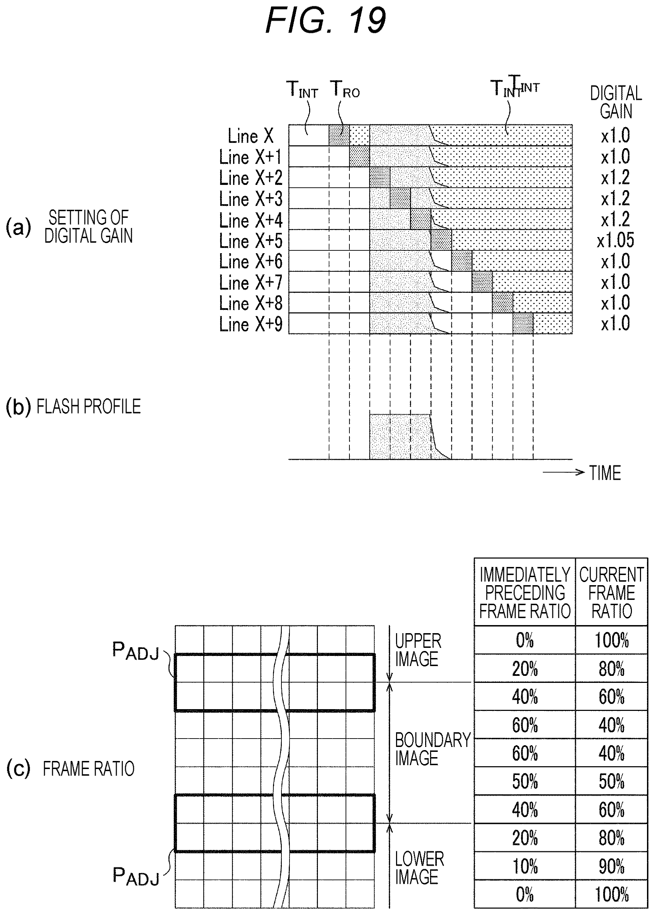

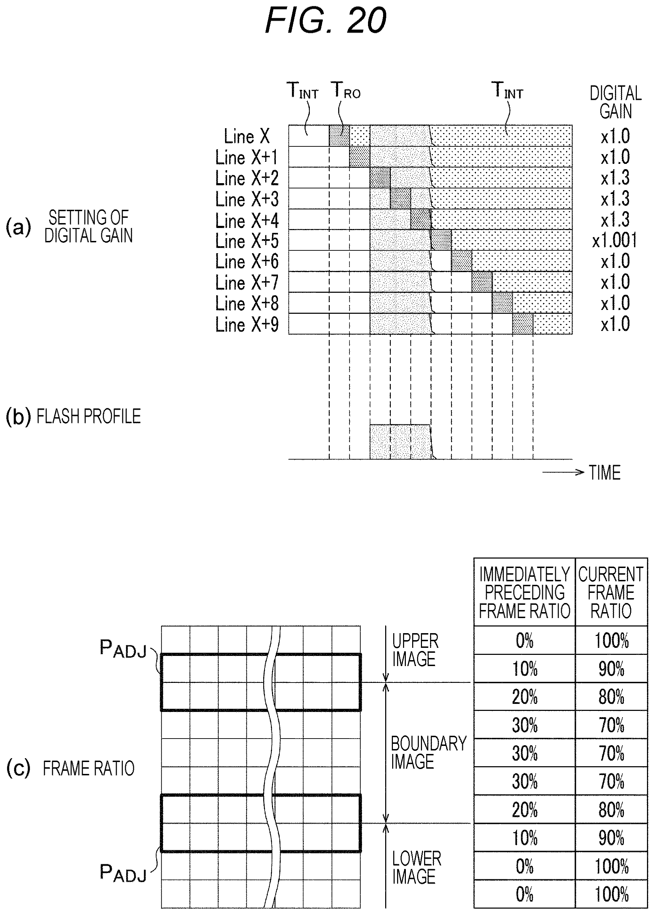

Next, a specific setting example in the second aspect for the post-processing on the composite image will be described with reference to Figs. 18 to 20.

In each of Figs. 18 to 20, (a) illustrates the setting of the digital gain for each pixel of each line of the boundary image, (b) illustrates the flash profile, and (c) illustrates the immediately preceding frame ratio and the current frame ratio in the interpolation processing. Note that (a) and (b) of Figs. 18 to 20 are the same as (a) and (b) of Figs. 11 to 13, respectively.

In (c) of Figs. 18 to 20, the "current frame ratio" is a weight (or ratio) (%) of the pixel value of the target pixel included in the composite image of the current frame that is the basis of the frame interpolation with respect to all the pixel values. The "immediately preceding frame ratio" is a weight (or ratio) (%) of the pixel value of the target pixel in the captured image of the immediately preceding frame that is the basis of the frame interpolation with respect to all the pixel values.

Here, the "immediately preceding frame" is a frame two frames before the current frame in a case where the flash is emitted at any line of the CMOS image sensor 14 at the exposure start time that is one frame before the current frame, and is a frame three frames before the current frame in a case where the flash is not emitted at any line of the CMOS image sensor 14 at the exposure start time that is one frame before the current frame.

In the embodiment, the interpolation processing unit 53 sets the weight of the weighted average such that the weight for the pixel values of the corresponding pixels in the captured image of the past frame (that is, two frames earlier or three frames earlier) is maximized at the center in the line direction of the boundary image, and the weight for the pixel values of the corresponding pixels in the captured image of the past frame decreases as the distance from the center increases in the vertical direction. For example, Fig. 18(c) illustrates an example in which the immediately preceding frame ratio is set to be maximum (80%) at the center in the line direction of the boundary image, and the immediately preceding frame ratio is set to decrease as the distance from the center increases in the vertical direction.

The reason for setting the immediately preceding frame ratio in this manner is as follows. That is, in general, since the flash profile has characteristics in which the emission intensity gradually rises from the start of emission and the emission intensity gradually falls toward the end of the emission, in order to compensate for the decrease in the exposure amount at the center where the emission intensity is the highest in the flash profile, for example, as illustrated in Fig. 18(a), the set digital gain is the highest for the line (that is, the center line of the boundary image) corresponding to the center of the flash profile. At this time, noise is also amplified at the center line of the boundary image by increasing the digital gain. Therefore, in order to reduce the sense of discomfort that the amplified noise gives to the observer, the immediately preceding frame ratio is set to be larger toward the center of the boundary image.

In the embodiment, the interpolation processing unit 53 may set the weight of the weighted average such that the weight for the pixel values of the pixels included in the line at the adjacent position where the boundary image and the upper image are adjacent to each other and/or the line at the adjacent position where the boundary image and the lower image are adjacent to each other is maximized, and the weight for the pixel values of the corresponding pixels of the captured image of the past frame (that is, two frames earlier or three frames earlier) decreases as the distance from the adjacent position increases in the vertical direction.

In the embodiment, the interpolation processing unit 53 sets the weight of the weighted average applied to the pixels included in the target line for the interpolation processing according to the flash profile.

Figs. 18 to 20 illustrate different flash profiles, and according to the flash profiles, the immediately preceding frame ratio and the current frame ratio illustrated in (c) of Figs. 18 to 20 are different. By adjusting the ratio according to the flash profile, it is possible to generate a composite image without the sense of discomfort.

As described above, the electronic endoscope system 1 includes an electronic scope 10 including the CMOS image sensor 14 configured to image an object using the rolling shutter method, and a processor 20 including the light source device 28 that emits a flash to perform strobe imaging of the object. The light source device 28 emits a flash such that a period from the flash end time of a certain flash to the flash start time of the next flash is longer than the flash prohibition period in which the emission of the flash is prohibited for at least one frame period. The processor 20 processes the captured image for each frame obtained by the CMOS image sensor 14 on the basis of the emission timing of the flash from the light source device 28 to generate a composite image, and generates screen data for monitor display on the basis of the composite image.

That is, in the electronic endoscope system 1, a flash prohibition period in which the emission of a flash is prohibited for at least one frame period is set. The length of the flash exposure period, which is an emission period of a single flash, can be freely set as long as the length of the flash prohibition period, which is an interval between the flashes, is equal to or longer than one frame period. Therefore, the stroboscopy can be implemented using a sufficient light intensity.

As illustrated in Figs. 3 to 7, the electronic endoscope system 1 allows the flash to be emitted at any line of the exposure start time of the CMOS image sensor 14 depending on the emission timing of the flash. Therefore, it is possible to suppress a decrease in the frame rate, and it is not necessary to cause the light source device 28 to emit the light in synchronization with the synchronization signal (Vsync or the like) of the CMOS image sensor 14.

On the other hand, when the composite image is generated, the upper image and lower image obtained by the CMOS image sensor 14 are combined. Therefore, a horizontal black line corresponding to the boundary image that is a boundary between the upper image and the lower image may appear. The horizontal black line can be made inconspicuous by performing at least one of digital gain application processing, spatial filter processing, or frame interpolation processing on the boundary image.

Note that, unlike the present embodiment, in a case where the CMOS image sensor is operated using a pseudo-global shutter method in which a common exposure period is set for all lines within one frame period, image synthesis processing is not necessary. However, the reading speed in the CMOS image sensor is forced to be decreased, and the decrease in the frame rate cannot be suppressed.

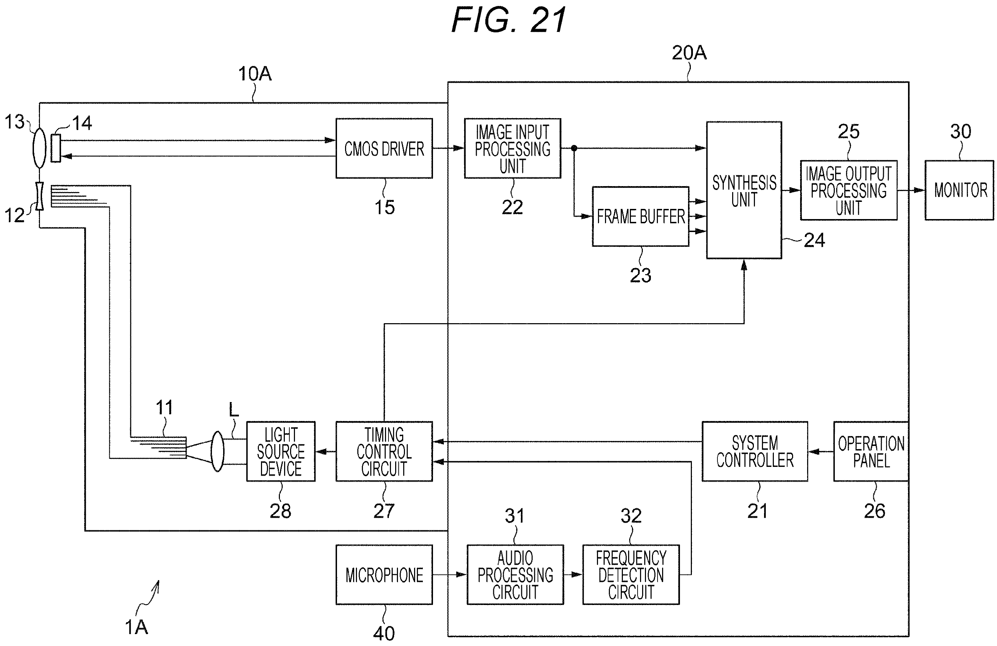

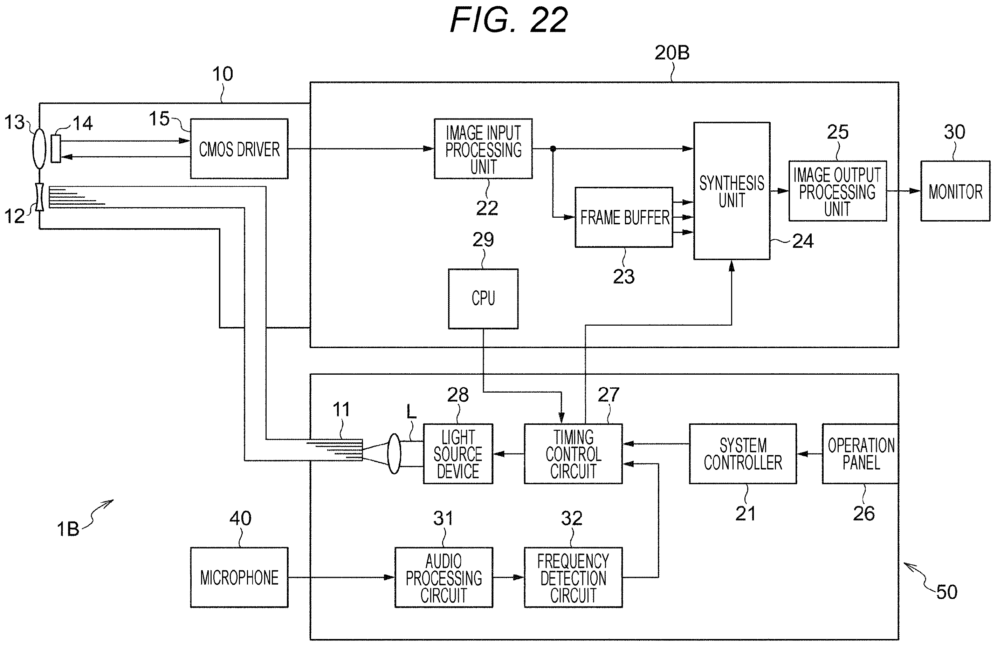

In the electronic endoscope system 1 illustrated in Fig. 1, since the light source device 28 is incorporated in the processor 20, there is an advantage that the processing of transmitting the emission timing signal from the timing control circuit 27 to the synthesis unit 24 is facilitated, and other configurations can be adopted as illustrated in Figs. 21 and 22. In each of Figs. 21 and 22, the same components as those included in the system of Fig. 1 are denoted by the same reference numerals.

An electronic endoscope system 1A of Fig. 21 implements an embodiment in a case where a light source device is incorporated in an electronic scope. As illustrated in Fig. 21, the electronic endoscope system 1A includes an electronic scope 10A and a processor 20B, and the electronic scope 10A is different from the electronic scope 10 illustrated in Fig. 1 in including a timing control circuit 27 and a light source device 28. In this configuration, the electronic scope 10A can set the length of the flash prohibition period on the basis of the frame period set by itself.

An electronic endoscope system 1B of Fig. 22 implements an embodiment in a case where an electronic scope, a processor, and a light source device are separate devices. As illustrated in Fig. 22, the electronic endoscope system 1B includes an electronic scope 10, a processor 20B, and a light source system 50. In this system, the processor 20B includes a CPU 29 that notifies the timing control circuit 27 of the light source system 50 of data related to the length of the emission prohibition period. The CPU 29 is only required to notify the data only once when the electronic endoscope system 1B is activated.

The description has been given in detail about the imaging system and the electronic endoscope system according to the present invention. However, the imaging system and the electronic endoscope system according to the present invention are not limited to the above embodiments, and may of course be modified and changed in various ways without departing from the gist of the present invention.

The present invention relates to a patent application of Japanese Patent Application No. 2023-4384 filed with the Japan Patent Office on January 16, 2023, the entire contents of which are incorporated herein by reference.

## Claims

1. An imaging system comprising:
an image sensor that images an object using a rolling shutter method;
a light source unit that emits a flash to perform strobe imaging of the object, the light source unit emitting the flash such that a period from a flash end time of a certain flash to a flash start time of a next flash is longer than a flash prohibition period during which emission of the flash is prohibited for at least one frame period; and
an image processing unit that processes a captured image for each frame obtained by the image sensor on a basis of an emission timing of the flash from the light source unit to generate a display image,
wherein in a case where the flash is emitted at any line of the image sensor at an exposure start time that is one frame before a current frame,
the image processing unit generates the display image of the current frame by
setting an upper image corresponding to lines above a flash start line that is one frame earlier in the display image of the current frame as a captured image obtained from corresponding lines of the current frame,
setting a lower image corresponding to lines below a flash end line that is one frame earlier in the display image of the current frame as a captured image obtained from corresponding lines that are one frame earlier, and
setting a boundary image from the flash start line to the flash end line in the display image of the current frame as an image obtained by adding the captured image obtained from the corresponding lines of the current frame and the captured image obtained from the corresponding lines that are one frame earlier, and
in a case where the flash is not emitted at any line of the image sensor at the exposure start time that is one frame before the current frame,
the image processing unit generates the display image of the current frame by
setting an upper image corresponding to lines above the flash start line that is two frames earlier in the display image of the current frame as a captured image obtained from corresponding lines that are one frame earlier,
setting a lower image corresponding to lines below the flash end line that is two frames earlier in the display image of the current frame as a captured image obtained from corresponding lines that are two frames earlier, and
setting a boundary image from the flash start line to the flash end line in the display image of the current frame as an image obtained by adding the captured image obtained from corresponding lines that are one frame earlier and the captured image obtained from corresponding lines that are two frames earlier.

2. The imaging system according to claim 1, wherein
the image processing unit includes an amplification processing unit that amplifies a pixel value of each pixel of the display image of the current frame, and
the amplification processing unit sets a gain to be applied to pixels of each line of the boundary image in the display image of the current frame to be larger than a gain to be applied to pixels of each line of images other than the boundary image according to an emission intensity profile corresponding to elapse of time during the flash and a reading period of the image sensor.

3. The imaging system according to claim 1 or 2, wherein
the image processing unit includes a filter processing unit that applies a spatial filter to pixel values of pixels included in a predetermined number of upper and lower lines from an adjacent position where the boundary image and the upper image are adjacent to each other and a predetermined number of upper and lower lines from an adjacent position where the boundary image and the lower image are adjacent to each other, for the display image of the current frame.

4. The imaging system according to claim 1 or 2, wherein
the image processing unit includes a filter processing unit that applies a spatial filter to pixel values of pixels included in a predetermined number of upper and lower lines from a central position in a line direction of the boundary image, for the display image of the current frame.

5. The imaging system according to claim 3, wherein
the filter processing unit sets filter coefficients of the spatial filter applied to the pixels included in each line of the predetermined number of lines according to an emission intensity profile corresponding to elapse of time during the flash.

6. The imaging system according to claim 1 or 2, wherein
the image processing unit includes an interpolation processing unit that performs interpolation processing between a current frame and a past frame that is two or three frames before the current frame, using, as a target line, each line of the boundary image in the display image of the current frame, or each line of a plurality of lines including a predetermined number of lines above an adjacent position where the boundary image and the upper image are adjacent to each other and a predetermined number of lines below an adjacent position where the boundary image and the lower image are adjacent to each other, as well as each line of the boundary image,
wherein in a case where the flash is emitted at any line of the image sensor at the exposure start time that is one frame before the current frame, the interpolation processing unit calculates pixel values of pixels included in each target line in the display image of the current frame by performing weighted average on pixel values of corresponding pixels in the display image of the current frame and pixel values of corresponding pixels in a captured image that is two frames earlier, and
in a case where the flash is not emitted at any line of the image sensor at the exposure start time that is one frame before the current frame, the interpolation processing unit calculates the pixel values of the pixels included in each target line in the display image of the current frame by performing the weighted average on the pixel values of corresponding pixels in the display image of the current frame and the pixel values of corresponding pixels in the captured image that is three frames earlier.

7. The imaging system according to claim 6, wherein
the interpolation processing unit sets a weight of the weighted average such that the weight for the pixel values of corresponding pixels in the captured image of the past frame is maximized at a center in a line direction of the boundary image, and the weight for the pixel values of corresponding pixels in the captured image of the past frame decreases as a distance from the center increases in a vertical direction.

8. The imaging system according to claim 6, wherein
the interpolation processing unit sets a weight of the weighted average such that the weight for the pixel values of the pixels included in a line at the adjacent position where the boundary image and the upper image are adjacent to each other and/or a line at the adjacent position where the boundary image and the lower image are adjacent to each other is maximized, and the weight for the pixel values of the corresponding pixels of the captured image of the past frame decreases as a distance from the adjacent position increases in a vertical direction.

9. The imaging system according to claim 6, wherein
the interpolation processing unit sets a weight of the weighted average applied to the pixels included in the target line according to an emission intensity profile corresponding to elapse of time during the flash.

10. An electronic endoscope system comprising:
a microphone;
a voice detection unit that detects a voice frequency from an audio signal acquired from the microphone; and
the imaging system according to any one of claims 1 to 9,
wherein the light source unit emits the flash at a cycle synchronized with the voice frequency detected by the voice detection unit.
